# EUROPEAN PATENT SPECIFICATION

(11) **EP 1 713 909 B1**
(45) Date of publication and mention of the grant of the patent: **05.08.2009**
(21) Application number: 05796124.5
(22) Date of filing: 28.01.2005
(51) Int. Cl.: C12N 15/00

(54) **ALTERING ROOT STRUCTURE DURING PLANT DEVELOPMENT**
VERÄNDERUNG DER WURZELSTRUKTUR WÄHREND DER PFLANZENENTWICKLUNG
MODIFICATION DE LA STRUCTURE DES RACINES AU COURS DU DEVELOPPEMENT D'UN VEGETAL

(30) Priority: 02.02.2004 US 541142 P
(43) Date of publication of application: 25.10.2006
(73) Proprietor: E.I. DU PONT DE NEMOURS AND COMPANY, Wilmington, DE 19898 (US); Pioneer Hybrid International, Des Moines, IA 50309 (US)
(72) Inventor: TARAMINO, Graziana, Wilmington, Delaware 19805 (US); SAKI, Hajime, Newark, Delaware 19711 (US); MEELEY, Robert, B., Des Moines, Iowa 50312 (US); NIU, Xiaomu, Johnson, Iowa 50131 (US)
(74) Representative: Webber, Philip Michael
(86) International application number: PCT/US2005/003332
(87) International publication number: WO 2006/006956

(56) References cited:
- SHUAI BIN ET AL: "The LATERAL ORGAN BOUNDARIES gene defines a novel, plant-specific gene family" PLANT PHYSIOLOGY (ROCKVILLE), vol. 129, no. 2, June 2002 (2002-06), pages 747-761, XP002366088 ISSN: 0032-0889 cited in the application
- HOCHHOLDINGER FRANK ET AL: "From weeds to crops: Genetic analysis of root development in cereals." TRENDS IN PLANT SCIENCE, vol. 9, no. 1, January 2004 (2004-01), pages 42-48, XP002366089 ISSN: 1360-1385
- HETZ WINFRIED ET AL: "Isolation and characterization of rtcs, a maize mutant deficient in the formation of nodal roots" PLANT JOURNAL, BLACKWELL SCIENTIFIC PUBLICATIONS, OXFORD, GB, vol. 10, no. 5, 1996, pages 845-857, XP002182550 ISSN: 0960-7412 cited in the application
- HOCHHOLDINGER FRANK ET AL: "Genetic dissection of root formation in maize (Zea mays) reveals root-type specific developmental programmes" ANNALS OF BOTANY (LONDON), vol. 93, no. 4, April 2004 (2004-04), pages 359-368, XP002366090 ISSN: 0305-7364
- DATABASE UniProt [Online] 1 March 2003 (2003-03-01), "Hypothetical protein OSJNBb0050N02.10." XP002369174 retrieved from EBI accession no. UNIPROT:Q8H081 Database accession no. Q8H081 cited in the application

### SEQUENCE LISTING

<110> Taramino, Graziana
   Sakai, Hajime
   Meeley, Robert B.
   Niu, Xiaomu
<120> ALTERING ROOT STRUCTURE DURING PLANT DEVELOPMENT
   <130> BB1545
<160> 42
<170> PatentIn version 3.2
<210> 1
   <211> 6101
   <212> DNA
   <213> Zea Maize
<400> 1
   acctcgcaag tttttccagt tttttttaaa gaccacctca caagctgctt tttccaatct     60
   gaacaattga acatgtagta acaataaata aacaggtcag agttcttccc gctccagttt    120
   ccgatgctcg acagattaac gctagttccc agaggatgag caaaccggca ggattaaaca    180
   aacgatgggg gagagaaaga gctgcattag tggtcatcgt gggggccaat agagtcgaga    240
   atactaaagc acctggggcc tggggggtac gtacccgaac taccctagcc tttctttctt    300
   tctttccatc tcactatttt ttgccacctc ctatacacac acatggtggg cttgatgtga    360
   gaggatgccc ggggcaaaca acacgtaaaa actaccaagg agatccagcc taataaagct    420
   cgggaaaatt tagaagggca tccaacccaa ccaggtagtg tttctgaaac tagtgacagc    480
   cctaccctag cgctagcgaa tccagagaaa tccacgtact gcttcttttc ttacggaaac    540
   cgtagggact tttttgcatt gttttcttat tcttcttctt cttgagatag gagtagggat    600
   gtactccatc cagttagttt tgtaagaggc taatgttaaa agtagaaata tcaaaaactt    660
   cgtataattt gaaaagttaa aagtagttat tgcccaaatt actagctaaa gattgtgacg    720
   ttcggatttg ctatgactag agggagcatt aatataacat ataatgttgt atgcaaattt    780
   taaaaagttt caaaacaggg tcagaacaag gaatggccaa attaaatgac taacttagct    840
   tgttaattat ctatgggtgt aatttactgg ctttcgagat ttgttattac gggtgtaaaa    900
   cgtactgaat tgagaaactg acgtgctagc tcttgacagt gtaaacgcgt catcagtagt    960
   tcctacgcat cattatcagg ggctggctag ctgctgagct gctatttgct atgcatgttt   1020
   ctgctagcag ttacaagccc taaactggaa aatgccggtc tatagcaccc cagctgacga   1080
   ccagaaatag gcgatgaagc tattgcgttc tttgccctaa aaaaagaact taaaaaaggt   1140
   tattgtatcg tccacatgac aggtagtaaa agtgacctgt attttttttc tcataaaaaa    1200
   tgtgaccttt tgctcttgct ttgaaggcgt aataactagg aacgaacaac aagaggcagg   1260
   gtgcatgtag ttgttgcagt tgcatctcaa tggaagccct caatcatgag agcatgaaca   1320
   catcactgct cattgtcatt ccttccattc catccatgtt tggacagatg aaggaagagg   1380
   ccacagctga aggctgaggc cagcactcct tcccatctgt ctttgttact aattacctgg   1440
   gcctatgaat ctaggatgca gtagactgat ggatgtttac aaattacaat gccatcaatg   1500
   atgatgccca ggatatgcta gttatggaat caacataata acgcaatgat aagtaagcat   1560
   aggccatgac cagccatcta ataacagatt agattaatta ggacagagta gactaatctg   1620
   gttgagcaaa cacttccagt ggctagtgga aggcaaaaag cctgattagg atatacaact   1680
   acaggcgcca gtacgtacta atcgtccttt tgagcattct gtgaagcaga acagaggcgg   1740
   cagagtttca gaagttctgc tgccctgttc cagtccttca taggtgcaac tgctatacta   1800
   cacgaacaaa cagtacatct tcagctatac taaattcagt tttttttctt cttacaaacg   1860
   catattttaa gctacagcat tggcagtcca ttgctcgatt tgtttttttc tcggtggttg   1920
   gacttacatg cctacaggaa aactaaaaca atacgtatat gtggttttct gataatcaaa   1980
   tcaaagcggg gggatgtgac accagaacta gttctttccc atcaccccat tattgtttgc   2040
   ttttgcccag tctcgcgaag aaaaaatgaa atcaaaagaa aatatcaaag cgaggagcag   2100
   cgacaactcc acgtctggag ccaggtgatg tatgagtgca ggtactacac ggtacataga   2160
   ttttattttt tttaaaaaaa acacataagc atttatttta tttatcccca aattatgaac   2220
   tggactttgc tcgctggtct cgcagcagcc gagcccaact gcacacaaaa gaaatgggcg   2280
   catgagcagg cacagaaaaa ataaacagag aaagcatgca ttaattagac caaacccaaa   2340
   acccctaagc aaaagattag caatgattgg cgtctccatt gtgcacttgc acaggtacta   2400
   gtactcctgc taggcttgtt gttgtagggt gcctgcccca tgcagtgcaa ggagggaggg   2460
   gtgtgtcacc ataaaaattt agcggcaagg gcgagggcga ttggaagctc aaaataatga   2520
   gctggttgcc caccggggag acacgccgga tttgtttaat cccctggccc taatccccca   2580
   gccctgccgt ctcctcctta taagcaatgg cggaggggtc ttgctcttgc attgcacctc   2640
   cggccaccgc gccatatata gccgcagtaa gcaggcgaga acgacgaaga ggtcacgcac   2700
   cacaccgatc aatccagctc gagcgaccga tcacgtgcag cacagcacag cacagcggtg   2760
   ctcgatcggc gaagagagat gacggggttc gggtcaccgt gcggggcgtg caagttcctg   2820
   cgccgcaagt gcgtgcgcgg ctgcgtcttc gcgccctact tctgccacga gcagggcgcg   2880
   gcgcacttcg ccgccatcca caaggtgttc ggcgccagca acgtgtccaa gctgctcgcg   2940
   cacctgccgc tcgccgaccg cgccgaggcc gccgtcacca tctcctacga ggcgcaggcg   3000
   aggctacgcg accccatcta tggctgcgtc gcccacatct tcgcgctaca gcagcaggtg   3060
   tgcatgcgcg actgccccgg ccgccgcgcc gtctctgggc ttgtctcttg attgtgatag   3120
   ggtttaattg ctgaccggcc cgtgccaatc gatccaggtg atgaccctgc aggcgcagct   3180
   ggcgtcgctc aaggcgcagg cggcgcaggg gcagcagggc gtgcacgagg acgccaaggg   3240
   ctacgtgggc agcgccgccg cggagcagct aggttacggc tacccctggt gcagcggcaa  3300
   tggaggcgcc gcagcagcag caggcgccgt gggcgcgccc gccgcgcagc cgggcgcgta   3360
   cggcaatggc gcgcacgagt ccctgaccgc gctgctgggg tcgtcggact acatgcagca   3420
   gtcgctgtac cacgcgttcg agcaggccgg cgcggacgac gacgacggcc ggcaggggta   3480
   cggcttcgag gcagcggcgg agtcctcgtc gctcggggcg gaggagagcg ggtggaggtc   3540
   gtcgtcgggg taccaagact gcgaggacct gcagagcgtg gcttacgctt acctgaacca   3600
   tcgctcgtaa gaactgagaa ctactactac tacaagagag agagagagag agatagatat   3660
   atagacatat ctgtcctcaa ttcctgatca tgttttggac tttagcctgg ggaaatatat   3720
   gcgcgatttt cgatcgatca gtcgatcggt ctccgctaca aataatccag aagcatgcat   3780
   gcatgtgaca gaccactgat atataataga tccacacatt gatcatcatc agtgtagaaa   3840
   ttaacgtacg tagcctaatt aattggacaa agaaaaaaat gaagagccct tgctgtgatt   3900
   atgctgctag ttctgtcagg ggtggggttg tgttttcttc tccaactctc tgcctacctg   3960
   ctgcagcagt gtctgcagac gataaggtta gattcgtcat gccggccgga aaatgtactc   4020
   caaggaacat acaaggcagc atattgagag acaggtgatt gattcatggc cacacgtgga   4080
   agatccaatt agcctactat tttcgttgac tccttttact ggaactcttt ctgatgggac   4140
   atgcacacat cttcagcata tatatagcta ctagtagatg atatgataga gccttttgtc   4200
   ttgtgtagac aatcctacta tagtgattaa taactctcct atataactct cctatatctg   4260
   gattattatt accctatagc tacttgatta acacacatgt agatattcta aatcatgacc   4320
   attacatact taaaaaggga taattatggc gactcatcat aatttgtgtc gtgtctaata   4380
   atctgtctac tttttttccc aggactacat ttggtaaaaa tctttttctg aagtttttgt  4440
   ttatttgtaa cacaaatttt ggttagatgt ttccattgcg tagaccacca tcttttaaat   4500
   catagatgac tgtttcacac aaaaaaaatt gtggctagct agtgtaagtg taactatgaa   4560
   catatataat cttaaggcca gaaaaaaatt ctcttaaaac aggccagtcc tgaacgggtt   4620
   acatggacag caggatggaa caagcagctc atagttatat gggagataga accatgggat   4680
   atcaacctca acttgtgtgt gtaaatatat atggagcagg tcaaatgaca ttgttgcatg   4740
   aatacaaaca tgtatgaggt gtttgagttg atccatcgtc acctcatttc tcatagttag   4800
   ttagtactaa tattaggaat gaggtcatcc caccaaattt aaggaataaa ctcatatgat   4860
   gcaccacctc aatctggata gagtgattac tcaaaccaaa cacccccata gttacctagg   4920
   gtaaggatta tatacaatac atttgaatgg attgagcata acgaataagg gtgaatcgcc   4980
   atccataaaa tgtactagtt gcatgcttgc attagctagg atatattgat tatgatttat   5040
   gaagaggctt aggggctgtt tggtacggtt gccgcttggc agacaaaagc ctgacctcct   5100
   gtaatggttg ataatttgaa gtgcattatt tgtaaaaaaa aaatccatct tttgtggaac   5160
   agatccaaaa ctaattcttt gaaaaaaatt atataatccc aagaagctag cgtaaaatgg   5220
   accttatagt cattactcac ttaaacgcgg acataattca tttgacttct tgatgaatga   5280
   ctgtattgta tatgactcaa gaccactggt agatttacat actgtcttat taggccttgt   5340
   tcggttaatc ctgttagcca ttgattaaat gagattgaaa aaaaataaga gaaagtttga   5400
   cttgcttggg atttaaactc acccaatccc actcaatcca catggattga gaggtgtaaa   5460
   atcagaaaca gttcaactaa agtggtggca gaaacttgtt tccagaaatg ctaataaata   5520
   ggtttataca tgttgcacaa agtgtatttt tgtaaatcgg tacatgattc acaggtcgtt   5580
   gatttttttg cgggggtgtg gaattaacac cgtgtataca gcatggaaag caaaaaaata   5640
   caagaaaaag ctatggacga tgtcactttg catcctaatc aatagcacat gcatcaaagg   5700
   aaacttaata cgatatcata ccatacagga gatgagggca tcaattattg tcgtgtttaa   5760
   agtgaagaag gacacaagct ttttttttca tttatatgct tccatctgct caatatattt   5820
   tatatacagg ttaatgaaat atatctatat agatatatga tacaatgaac agcactacat   5880
   acatatatct gtcatgtcag aaaaatgaat gccaaaaaat aaattttctc ccattctaaa   5940
   taattgttga ttctcaggaa tactatctac aaataccaga tgacggactt gtctagattc   6000
   cagacccaac cgaatgcaaa ttaacttgac agacatatat ataagtaaag atgagcactg   6060
   catatatgtc ttgaagcgat ttggctgttg gagtggtcga t                       6101
<210> 2
   <211> 2778
   <212> DNA
   <213> Zea maize
<220>
   <221> promoter
   <222> (1)..(2778)
<400> 2
   acctcgcaag tttttccagt tttttttaaa gaccacctca caagctgctt tttccaatct 60
   gaacaattga acatgtagta acaataaata aacaggtcag agttcttccc gctccagttt 120
   ccgatgctcg acagattaac gctagttccc agaggatgag caaaccggca ggattaaaca    180
   aacgatgggg gagagaaaga gctgcattag tggtcatcgt gggggccaat agagtcgaga    240
   atactaaagc acctggggcc tggggggtac gtacccgaac taccctagcc tttctttctt    300
   tctttccatc tcactatttt ttgccacctc ctatacacac acatggtggg cttgatgtga    360
   gaggatgccc ggggcaaaca acacgtaaaa actaccaagg agatccagcc taataaagct    420
   cgggaaaatt tagaagggca tccaacccaa ccaggtagtg tttctgaaac tagtgacagc    480
   cctaccctag cgctagcgaa tccagagaaa tccacgtact gcttcttttc ttacggaaac    540
   cgtagggact tttttgcatt gttttcttat tcttcttctt cttgagatag gagtagggat    600
   gtactccatc cagttagttt tgtaagaggc taatgttaaa agtagaaata tcaaaaactt    660
   cgtataattt gaaaagttaa aagtagttat tgcccaaatt actagctaaa gattgtgacg    720
   ttcggatttg ctatgactag agggagcatt aatataacat ataatgttgt atgcaaattt    780
   taaaaagttt caaaacaggg tcagaacaag gaatggccaa attaaatgac taacttagct    840
   tgttaattat ctatgggtgt aatttactgg ctttcgagat ttgttattac gggtgtaaaa    900
   cgtactgaat tgagaaactg acgtgctagc tcttgacagt gtaaacgcgt catcagtagt    960
   tcctacgcat cattatcagg ggctggctag ctgctgagct gctatttgct atgcatgttt   1020
   ctgctagcag ttacaagccc taaactggaa aatgccggtc tatagcaccc cagctgacga   1080
   ccagaaatag gcgatgaagc tattgcgttc tttgccctaa aaaaagaact taaaaaaggt   1140
   tattgtatcg tccacatgac aggtagtaaa agtgacctgt attttttttc tcataaaaaa    1200
   tgtgaccttt tgctcttgct ttgaaggcgt aataactagg aacgaacaac aagaggcagg   1260
   gtgcatgtag ttgttgcagt tgcatctcaa tggaagccct caatcatgag agcatgaaca   1320
   catcactgct cattgtcatt ccttccattc catccatgtt tggacagatg aaggaagagg   1380
   ccacagctga aggctgaggc cagcactcct tcccatctgt ctttgttact aattacctgg   1440
   gcctatgaat ctaggatgca gtagactgat ggatgtttac aaattacaat gccatcaatg   1500
   atgatgccca ggatatgcta gttatggaat caacataata acgcaatgat aagtaagcat   1560
   aggccatgac cagccatcta ataacagatt agattaatta ggacagagta gactaatctg   1620
   gttgagcaaa cacttccagt ggctagtgga aggcaaaaag cctgattagg atatacaact   1680
   acaggcgcca gtacgtacta atcgtccttt tgagcattct gtgaagcaga acagaggcgg   1740
   cagagtttca gaagttctgc tgccctgttc cagtccttca taggtgcaac tgctatacta   1800
   cacgaacaaa cagtacatct tcagctatac taaattcagt tttttttctt cttacaaacg    1860
   catattttaa gctacagcat tggcagtcca ttgctcgatt tgtttttttc tcggtggttg    1920
   gacttacatg cctacaggaa aactaaaaca atacgtatat gtggttttct gataatcaaa   1980
   tcaaagcggg gggatgtgac accagaacta gttctttccc atcaccccat tattgtttgc   2040
   ttttgcccag tctcgcgaag aaaaaatgaa atcaaaagaa aatatcaaag cgaggagcag   2100
   cgacaactcc acgtctggag ccaggtgatg tatgagtgca ggtactacac ggtacataga   2160
   ttttattttt tttaaaaaaa acacataagc atttatttta tttatcccca aattatgaac   2220
   tggactttgc tcgctggtct cgcagcagcc gagcccaact gcacacaaaa gaaatgggcg   2280
   catgagcagg cacagaaaaa ataaacagag aaagcatgca ttaattagac caaacccaaa   2340
   acccctaagc aaaagattag caatgattgg cgtctccatt gtgcacttgc acaggtacta   2400
   gtactcctgc taggcttgtt gttgtagggt gcctgcccca tgcagtgcaa ggagggaggg   2460
   gtgtgtcacc ataaaaattt agcggcaagg gcgagggcga ttggaagctc aaaataatga   2520
   gctggttgcc caccggggag acacgccgga tttgtttaat cccctggccc taatccccca   2580
   gccctgccgt ctcctcctta taagcaatgg cggaggggtc ttgctcttgc attgcacctc   2640
   cggccaccgc gccatatata gccgcagtaa gcaggcgaga acgacgaaga ggtcacgcac   2700
   cacaccgatc aatccagctc gagcgaccga tcacgtgcag cacagcacag cacagcggtg   2760
   ctcgatcggc gaagagag                                                 2778
<210> 3
   <211> 2000
   <212> DNA
   <213> Zea maize
<220>
   <221> promoter
   <222> (1)..(2000)
<400> 3
   tttaaaaagt ttcaaaacag ggtcagaaca aggaatggcc aaattaaatg actaacttag     60
   cttgttaatt atctatgggt gtaatttact ggctttcgag atttgttatt acgggtgtaa    120
   aacgtactga attgagaaac tgacgtgcta gctcttgaca gtgtaaacgc gtcatcagta    180
   gttcctacgc atcattatca ggggctggct agctgctgag ctgctatttg ctatgcatgt    240
   ttctgctagc agttacaagc cctaaactgg aaaatgccgg tctatagcac cccagctgac    300
   gaccagaaat aggcgatgaa gctattgcgt tctttgccct aaaaaaagaa cttaaaaaag    360
   gttattgtat cgtccacatg acaggtagta aaagtgacct gtattttttt tctcataaaa    420
   aatgtgacct tttgctcttg ctttgaaggc gtaataacta ggaacgaaca acaagaggca    480
   gggtgcatgt agttgttgca gttgcatctc aatggaagcc ctcaatcatg agagcatgaa    540
   cacatcactg ctcattgtca ttccttccat tccatccatg tttggacaga tgaaggaaga    600
   ggccacagct gaaggctgag gccagcactc cttcccatct gtctttgtta ctaattacct    660
   gggcctatga atctaggatg cagtagactg atggatgttt acaaattaca atgccatcaa    720
   tgatgatgcc caggatatgc tagttatgga atcaacataa taacgcaatg ataagtaagc    780
   ataggccatg accagccatc taataacaga ttagattaat taggacagag tagactaatc    840
   tggttgagca aacacttcca gtggctagtg gaaggcaaaa agcctgatta ggatatacaa    900
   ctacaggcgc cagtacgtac taatcgtcct tttgagcatt ctgtgaagca gaacagaggc    960
   ggcagagttt cagaagttct gctgccctgt tccagtcctt cataggtgca actgctatac   1020
   tacacgaaca aacagtacat cttcagctat actaaattca gttttttttc ttcttacaaa   1080
   cgcatatttt aagctacagc attggcagtc cattgctcga tttgtttttt tctcggtggt   1140
   tggacttaca tgcctacagg aaaactaaaa caatacgtat atgtggtttt ctgataatca   1200
   aatcaaagcg gggggatgtg acaccagaac tagttctttc ccatcacccc attattgttt   1260
   gcttttgccc agtctcgcga agaaaaaatg aaatcaaaag aaaatatcaa agcgaggagc   1320
   agcgacaact ccacgtctgg agccaggtga tgtatgagtg caggtactac acggtacata   1380
   gattttattt tttttaaaaa aaacacataa gcatttattt tatttatccc caaattatga   1440
   actggacttt gctcgctggt ctcgcagcag ccgagcccaa ctgcacacaa aagaaatggg   1500
   cgcatgagca ggcacagaaa aaataaacag agaaagcatg cattaattag accaaaccca   1560
   aaacccctaa gcaaaagatt agcaatgatt ggcgtctcca ttgtgcactt gcacaggtac   1620
   tagtactcct gctaggcttg ttgttgtagg gtgcctgccc catgcagtgc aaggagggag   1680
   gggtgtgtca ccataaaaat ttagcggcaa gggcgagggc gattggaagc tcaaaataat   1740
   gagctggttg cccaccgggg agacacgccg gatttgttta atcccctggc cctaatcccc   1800
   cagccctgcc gtctcctcct tataagcaat ggcggagggg tcttgctctt gcattgcacc   1860
   tccggccacc gcgccatata tagccgcagt aagcaggcga gaacgacgaa gaggtcacgc   1920
   accacaccga tcaatccagc tcgagcgacc gatcacgtgc agcacagcac agcacagcgg   1980
   tgctcgatcg gcgaagagag                                                2000
<210> 4
   <211> 1000
   <212> DNA
   <213> Zea maize
<220>
   <221> promoter
   <222> (1)..(1000)
<400> 4
   cataggtgca actgctatac tacacgaaca aacagtacat cttcagctat actaaattca      60
   gttttttttc ttcttacaaa cgcatatttt aagctacagc attggcagtc cattgctcga      120
   tttgtttttt tctcggtggt tggacttaca tgcctacagg aaaactaaaa caatacgtat     180
   atgtggtttt ctgataatca aatcaaagcg gggggatgtg acaccagaac tagttctttc     240
   ccatcacccc attattgttt gcttttgccc agtctcgcga agaaaaaatg aaatcaaaag     300
   aaaatatcaa agcgaggagc agcgacaact ccacgtctgg agccaggtga tgtatgagtg     360
   caggtactac acggtacata gattttattt tttttaaaaa aaacacataa gcatttattt     420
   tatttatccc caaattatga actggacttt gctcgctggt ctcgcagcag ccgagcccaa     480
   ctgcacacaa aagaaatggg cgcatgagca ggcacagaaa aaataaacag agaaagcatg     540
   cattaattag accaaaccca aaacccctaa gcaaaagatt agcaatgatt ggcgtctcca     600
   ttgtgcactt gcacaggtac tagtactcct gctaggcttg ttgttgtagg gtgcctgccc     660
   catgcagtgc aaggagggag gggtgtgtca ccataaaaat ttagcggcaa gggcgagggc     720
   gattggaagc tcaaaataat gagctggttg cccaccgggg agacacgccg gatttgttta     780
   atcccctggc cctaatcccc cagccctgcc gtctcctcct tataagcaat ggcggagggg     840
   tcttgctctt gcattgcacc tccggccacc gcgccatata tagccgcagt aagcaggcga     900
   gaacgacgaa gaggtcacgc accacaccga tcaatccagc tcgagcgacc gatcacgtgc     960
   agcacagcac agcacagcgg tgctcgatcg gcgaagagag                           1000
<210> 5
   <211> 732
   <212> DNA
   <213> Zea Maize
<220>
   <221> CDS
   <222> (1)..(732)
<400> 5
   atg acg ggg ttc ggg tca ccg tgc ggg gcg tgc aag ttc ctg cgc cgc      48
   Met Thr Gly Phe Gly Ser Pro Cys Gly Ala Cys Lys Phe Leu Arg Arg
   1               5                   10                  15
   aag tgc gtg cgc ggc tgc gtc ttc gcg ccc tac ttc tgc cac gag cag      96
   Lys Cys Val Arg Gly Cys Val Phe Ala Pro Tyr Phe Cys His Glu Gln
               20                  25                  30
   ggc gcg gcg cac ttc gcc gcc atc cac aag gtg ttc ggc gcc agc aac      144
   Gly Ala Ala His Phe Ala Ala Ile His Lys Val Phe Gly Ala Ser Asn
           35                  40                  45
   gtg tcc aag ctg ctc gcg cac ctg ccg ctc gcc gac cgc gcc gag gcc      192
   Val Ser Lys Leu Leu Ala His Leu Pro Leu Ala Asp Arg Ala Glu Ala
       50                  55                  60
   gcc gtc acc atc tcc tac gag gcg cag gcg agg cta cgc gac ccc atc      240
   Ala Val Thr Ile Ser Tyr Glu Ala Gln Ala Arg Leu Arg Asp Pro Ile
   65                  70                  75                  80
   tat ggc tgc gtc gcc cac atc ttc gcg cta cag cag cag gtg atg acc      288
   Tyr Gly Cys Val Ala His Ile Phe Ala Leu Gln Gln Gln Val Met Thr
                   85                  90                  95
   ctg cag gcg cag ctg gcg tcg ctc aag gcg cag gcg gcg cag ggg cag      336
   Leu Gln Ala Gln Leu Ala Ser Leu Lys Ala Gln Ala Ala Gln Gly Gln
               100                 105                 110
   cag ggc gtg cac gag gac gcc aag ggc tac gtg ggc agc gcc gcc gcg      384
   Gln Gly Val His Glu Asp Ala Lys Gly Tyr Val Gly Ser Ala Ala Ala
           115                 120                 125
   gag cag cta ggt tac ggc tac ccc tgg tgc agc ggc aat gga ggc gcc      432
   Glu Gln Leu Gly Tyr Gly Tyr Pro Trp Cys Ser Gly Asn Gly Gly Ala
       130                 135                 140
   gca gca gca gca ggc gcc gtg ggc gcg ccc gcc gcg cag ccg ggc gcg      480
   Ala Ala Ala Ala Gly Ala Val Gly Ala Pro Ala Ala Gln Pro Gly Ala
   145                 150                 155                 160
   tac ggc aat ggc gcg cac gag tcc ctg acc gcg ctg ctg ggg tcg tcg      528
   Tyr Gly Asn Gly Ala His Glu Ser Leu Thr Ala Leu Leu Gly Ser Ser
                   165                 170                 175
   gac tac atg cag cag tcg ctg tac cac gcg ttc gag cag gcc ggc gcg      576
   Asp Tyr Met Gln Gln Ser Leu Tyr His Ala Phe Glu Gln Ala Gly Ala
               180                 185                 190
   gac gac gac gac ggc cgg cag ggg tac ggc ttc gag gca gcg gcg gag      624
   Asp Asp Asp Asp Gly Arg Gln Gly Tyr Gly Phe Glu Ala Ala Ala Glu
           195                 200                 205
   tcc tcg tcg ctc ggg gcg gag gag agc ggg tgg agg tcg tcg tcg ggg      672
   Ser Ser Ser Leu Gly Ala Glu Glu Ser Gly Trp Arg Ser Ser Ser Gly
       210                 215                 220
   tac caa gac tgc gag gac ctg cag agc gtg gct tac gct tac ctg aac      720
   Tyr Gln Asp Cys Glu Asp Leu Gln Ser Val Ala Tyr Ala Tyr Leu Asn
   225                 230                 235                 240
   cat cgc tcg taa                                                      732
   His Arg Ser
<210> 6
   <211> 243
   <212> PRT
   <213> Zea Maize
<400> 6
   Met Thr Gly Phe Gly Ser Pro Cys Gly Ala Cys Lys Phe Leu Arg Arg
   1               5                   10                  15
   Lys Cys Val Arg Gly Cys Val Phe Ala Pro Tyr Phe Cys His Glu Gln
               20                  25                  30
   Gly Ala Ala His Phe Ala Ala Ile His Lys Val Phe Gly Ala Ser Asn
           35                  40                  45
   Val Ser Lys Leu Leu Ala His Leu Pro Leu Ala Asp Arg Ala Glu Ala
       50                  55                  60
   Ala Val Thr Ile Ser Tyr Glu Ala Gln Ala Arg Leu Arg Asp Pro Ile
   65                  70                  75                  80
   Tyr Gly Cys Val Ala His Ile Phe Ala Leu Gln Gln Gln Val Met Thr
                   85                  90                  95
   Leu Gln Ala Gln Leu Ala Ser Leu Lys Ala Gln Ala Ala Gln Gly Gln
               100                 105                 110
   Gln Gly Val His Glu Asp Ala Lys Gly Tyr Val Gly Ser Ala Ala Ala
           115                 120                 125
   Glu Gln Leu Gly Tyr Gly Tyr Pro Trp Cys Ser Gly Asn Gly Gly Ala
       130                 135                 140
   Ala Ala Ala Ala Gly Ala Val Gly Ala Pro Ala Ala Gln Pro Gly Ala
   145                 150                 155                 160
   Tyr Gly Asn Gly Ala His Glu Ser Leu Thr Ala Leu Leu Gly Ser Ser
                   165                 170                 175
   Asp Tyr Met Gln Gln Ser Leu Tyr His Ala Phe Glu Gln Ala Gly Ala
               180                 185                 190
   Asp Asp Asp Asp Gly Arg Gln Gly Tyr Gly Phe Glu Ala Ala Ala Glu
           195                 200                 205
   Ser Ser Ser Leu Gly Ala Glu Glu Ser Gly Trp Arg Ser Ser Ser Gly
       210                 215                 220
   Tyr Gln Asp Cys Glu Asp Leu Gln Ser Val Ala Tyr Ala Tyr Leu Asn
   225                 230                 235                 240
   His Arg Ser
<210> 7
   <211> 780
   <212> DNA
   <213> Oryza sativa
<220>
   <221> CDS
   <222> (1)..(780)
<400> 7
   atg acg gga ttt gga tcg ccg tgc ggc gcg tgc aag ttt ctg cgg cgc      48
   Met Thr Gly Phe Gly Ser Pro Cys Gly Ala Cys Lys Phe Leu Arg Arg
   1               5                   10                  15
   aag tgc gtg cgc ggg tgc gtg ttc gcg cca tac ttc tgc cac gag caa      96
   Lys Cys Val Arg Gly Cys Val Phe Ala Pro Tyr Phe Cys His Glu Gln
               20                  25                  30
   ggg gcg gcg cac ttc gcc gcc atc cac aag gtg ttc ggc gcc agc aac      144
   Gly Ala Ala His Phe Ala Ala Ile His Lys Val Phe Gly Ala Ser Asn
           35                  40                  45
   gtg tcc aag ctg ctc gcc cac ctg ccg ctc gcc gac cgc ccc gag gcc      192
   Val Ser Lys Leu Leu Ala His Leu Pro Leu Ala Asp Arg Pro Glu Ala
       50                  55                  60
   gcc gtc act atc tcc tac gag gcg cag gcc cgc ctc cgc gac ccc atc      240
   Ala Val Thr Ile Ser Tyr Glu Ala Gln Ala Arg Leu Arg Asp Pro Ile
   65                  70                  75                  80
   tat ggc tgc gtc gcc cac atc ttc gcc ctc cag cag cag gtt atg acg      288
   Tyr Gly Cys Val Ala His Ile Phe Ala Leu Gln Gln Gln Val Met Thr
                   85                  90                  95
   ctg cag gcg cag ctg gcg tcg ctc aag gcg gcg gcg gcg caa ggg ata      336
   Leu Gln Ala Gln Leu Ala Ser Leu Lys Ala Ala Ala Ala Gln Gly Ile
               100                 105                 110
   cac cac cag gac gtc ggc gcc acc acc aag ggc ggc tac atg agc gcc      384
   His His Gln Asp Val Gly Ala Thr Thr Lys Gly Gly Tyr Met Ser Ala
           115                 120                 125
   gcc gcc acc gcc gcc gac gac caa tta ggg tac ggc ggc tac aac cag      432
   Ala Ala Thr Ala Ala Asp Asp Gln Leu Gly Tyr Gly Gly Tyr Asn Gln
       130                 135                 140
   tgg tgc ggc agc aat ggg ggc ggc gcg ccg gcg gcg tcg cag ccg ggc      480
   Trp Cys Gly Ser Asn Gly Gly Gly Ala Pro Ala Ala Ser Gln Pro Gly
   145                 150                 155                 160
   gcg tat agc agc aat ggc ggc gcc ggc cac ggc cac gac tcc atc acc      528
   Ala Tyr Ser Ser Asn Gly Gly Ala Gly His Gly His Asp Ser Ile Thr
                   165                 170                 175
   gcg ctg ctg gcg gcc ggg tcg gac tac atg cag cac tcg ctg tac cac      576
   Ala Leu Leu Ala Ala Gly Ser Asp Tyr Met Gln His Ser Leu Tyr His
               180                 185                 190
   gcg ttc gag cac tcg gag ggc gcc ggc gcc gtg gac gac ggg cac gcg      624
   Ala Phe Glu His Ser Glu Gly Ala Gly Ala Val Asp Asp Gly His Ala
           195                 200                 205
   gcc gcc gcg gcc ttc gag gcg gcg gcg gag tcg tcg tcg tgc ggc atg      672
   Ala Ala Ala Ala Phe Glu Ala Ala Ala Glu Ser Ser Ser Cys Gly Met
       210                 215                 220
   gcg gcg tcg ttc gcc gcc gac gag agc gtg tgg agg tcg tcg tcg tcg      720
   Ala Ala Ser Phe Ala Ala Asp Glu Ser Val Trp Arg Ser Ser Ser Ser
   225                 230                 235                 240
   gga tac caa gat tgc gag gat ctc cag agc gtc gcc tac gct tac ctt      768
   Gly Tyr Gln Asp Cys Glu Asp Leu Gln Ser Val Ala Tyr Ala Tyr Leu
                   245                 250                 255
   aac cgc tcg taa                                                      780
   Asn Arg Ser
<210> 8
   <211> 259
   <212> PRT
   <213> Oryza sativa
<400> 8
   Met Thr Gly Phe Gly Ser Pro Cys Gly Ala Cys Lys Phe Leu Arg Arg
   1               5                   10                  15
   Lys Cys Val Arg Gly Cys Val Phe Ala Pro Tyr Phe Cys His Glu Gln
               20                  25                  30
   Gly Ala Ala His Phe Ala Ala Ile His Lys Val Phe Gly Ala Ser Asn
           35                  40                  45
   Val Ser Lys Leu Leu Ala His Leu Pro Leu Ala Asp Arg Pro Glu Ala
       50                  55                  60
   Ala Val Thr Ile Ser Tyr Glu Ala Gln Ala Arg Leu Arg Asp Pro Ile
   65                  70                  75                  80
   Tyr Gly Cys Val Ala His Ile Phe Ala Leu Gln Gln Gln Val Met Thr
                   85                  90                  95
   Leu Gln Ala Gln Leu Ala Ser Leu Lys Ala Ala Ala Ala Gln Gly Ile
               100                 105                 110
   His His Gln Asp Val Gly Ala Thr Thr Lys Gly Gly Tyr Met Ser Ala
   115 120 125
   Ala Ala Thr Ala Ala Asp Asp Gln Leu Gly Tyr Gly Gly Tyr Asn Gln
   130 135 140
   Trp Cys Gly Ser Asn Gly Gly Gly Ala Pro Ala Ala Ser Gln Pro Gly
   145 150 155 160
   Ala Tyr Ser Ser Asn Gly Gly Ala Gly His Gly His Asp Ser Ile Thr
   165 170 175
   Ala Leu Leu Ala Ala Gly Ser Asp Tyr Met Gln His Ser Leu Tyr His
   180 185 190
   Ala Phe Glu His Ser Glu Gly Ala Gly Ala Val Asp Asp Gly His Ala
   195 200 205
   Ala Ala Ala Ala Phe Glu Ala Ala Ala Glu Ser Ser Ser Cys Gly Met
   210 215 220
   Ala Ala Ser Phe Ala Ala Asp Glu Ser Val Trp Arg Ser Ser Ser Ser
   225 230 235 240
   Gly Tyr Gln Asp Cys Glu Asp Leu Gln Ser Val Ala Tyr Ala Tyr Leu
   245 250 255
   Asn Arg Ser
<210> 9
   <211> 111
   <212> PRT
   <213> Zea Maize
<220>
   <221> DOMAIN
   <222> (1)..(111)
<220>
   <221> DOMAIN
   <222> (1)..(111)
   <223> Xaa=any amino acid
<220>
   <221> misc_feature
   <222> (62)..(62)
   <223> Xaa can be any naturally occurring amino acid
<220>
   <221> misc_feature
   <222> (107)..(107)
   <223> Xaa can be any naturally occurring amino acid
<400> 9
   Met Thr Gly Phe Gly Ser Pro Cys Gly Ala Cys Lys Phe Leu Arg Arg
   1               5                   10                  15
   Lys Cys Val Arg Gly Cys Val Phe Ala Pro Tyr Phe Cys His Glu Gln
               20                  25                  30
   Gly Ala Ala His Phe Ala Ala Ile His Lys Val Phe Gly Ala Ser Asn
           35                  40                  45
   Val Ser Lys Leu Leu Ala His Leu Pro Leu Ala Asp Arg Xaa Glu Ala
       50                  55                  60
   Ala Val Thr Ile Ser Tyr Glu Ala Gln Ala Arg Leu Arg Asp Pro Ile
   65                  70                  75                  80
   Tyr Gly Cys Val Ala His Ile Phe Ala Leu Gln Gln Gln Val Met Thr
                   85                  90                  95
   Leu Gln Ala Gln Leu Ala Ser Leu Lys Ala Xaa Ala Ala Gln Gly
               100                 105                 110
<210> 10
   <211> 21
   <212> PRT
   <213> Zea Maize
<220>
   <221> DOMAIN
   <222> (1)..(21)
   <223> Xaa = any amino acid
<220>
   <221> misc_feature
   <222> (6)..(6)
   <223> Xaa can be any naturally occurring amino acid
<220>
   <221> misc_feature
   <222> (8)..(8)
   <223> Xaa can be any naturally occurring amino acid
<220>
   <221> misc_feature
   <222> (12)..(13)
   <223> Xaa can be any naturally occurring amino acid
<220>
   <221> misc_feature
   <222> (17)..(17)
   <223> Xaa can be any naturally occurring amino acid
<220>
   <221> misc_feature
   <222> (19)..(19)
   <223> Xaa can be any naturally occurring amino acid
<400> 10
   Gln Leu Gly Tyr Gly Xaa Tyr Xaa Pro Trp Cys Xaa Xaa Asn Gly Gly
   1               5                   10                  15
   Xaa Ala Xaa Ala Ala
               20
<210> 11
   <211> 13
   <212> PRT
   <213> Zea maize
<220>
   <221> DOMAIN
   <222> (1)..(13)
   <223> Xaa=any amino acid
<220>
   <221> misc_feature
   <222> (6)..(6)
   <223> Xaa can be any naturally occurring amino acid
<400> 11
   Ser Asp Tyr Met Gln Xaa Ser Leu Tyr His Ala Phe Glu
   1               5                   10
<210> 12
   <211> 10
   <212> PRT
   <213> Zea maize
<220>
   <221> DOMAIN
   <222> (1)..(10)
   <223> Xaa=any amino acid
<400> 12
   Gly Phe Glu Ala Ala Ala Glu Ser Ser Ser
   1               5                   10
<210> 13
   <211> 28
   <212> PRT
   <213> Zea maize
<220>
   <221> DOMAIN
   <222> (1)..(28)
   <223> Xaa=any amino acid
<220>
   <221> misc_feature
   <222> (2)..(2)
   <223> Xaa can be any naturally occurring amino acid
<220>
   <221> misc_feature
   <222> (5)..(5)
   <223> Xaa can be any naturally occurring amino acid
<220>
   <221> misc_feature
   <222> (11)..(11)
   <223> Xaa can be any naturally occurring amino acid
<400> 13
   Ala Xaa Glu Ser Xaa Trp Arg Ser Ser Ser Xaa Gly Tyr Gln Asp Cys
   1               5                   10                  15
   Glu Asp Leu Gln Ser Val Ala Tyr Ala Tyr Leu Asn
               20                  25
<210> 14
   <211> 20
   <212> DNA
   <213> Zea maize
<220>
   <221> primer_bind
   <222> (1)..(20)
<400> 14
   cacgctgttt cagacaggaa 20
<210> 15
   <211> 20
   <212> DNA
   <213> Zea maize
<220>
   <221> primer_bind
   <222> (1)..(20)
<400> 15
   cgcctgtgat tgcactacac 20
<210> 16
   <211> 20
   <212> DNA
   <213> Zea maize
<220>
   <221> primer_bind
   <222> (1)..(20)
<400> 16
   ctcctcgcaa ggatcttcac 20
<210> 17
   <211> 20
   <212> DNA
   <213> Zea maize
<220>
   <221> primer_bind
   <222> (1)..(20)
<400> 17
   agcaccgttt ctcgtgagat 20
<210> 18
   <211> 24
   <212> DNA
   <213> Zea maize
<220>
   <221> primer_bind
   <222> (1)..(24)
<400> 18
   tagtttgagg gatcaagaac cacc 24
<210> 19
   <211> 24
   <212> DNA
   <213> Zea maize
<220>
   <221> primer_bind
   <222> (1)..(24)
<400> 19
   gctcaaaggc aaggcagtat ttta 24
<210> 20
   <211> 24
   <212> DNA
   <213> Zea maize
<220>
   <221> primer_bind
   <222> (1)..(24)
<400> 20
   cgtttgatat gatgtggaga ttcg 24
<210> 21
   <211> 24
   <212> DNA
   <213> Zea maize
<220>
   <221> primer_bind
   <222> (1)..(24)
<400> 21
   aagcttgtga atgttctgga tgtc 24
<210> 22
   <211> 20
   <212> DNA
   <213> Zea maize
<220>
   <221> primer_bind
   <222> (1)..(20)
<400> 22
   ggtgaaccct tttgaagcag 20
<210> 23
   <211> 20
   <212> DNA
   <213> Zea maize
<220>
   <221> primer_bind
   <222> (1)..(20)
<400> 23
   actggaacaa gaacgccatc 20
<210> 24
   <211> 23
   <212> DNA
   <213> Zea maize
<220>
   <221> primer_bind
   <222> (1)..(23)
<400> 24
   aatagcgcaa gctgctgttg tat 23
<210> 25
   <211> 23
   <212> DNA
   <213> Zea maize
<220>
   <221> primer_bind
   <222> (1)..(23)
<400> 25
   cccttgtcac tgtcgaaacc tac 23
<210> 26
   <211> 287
   <212> PRT
   <213> Oryza sativa
<220>
   <221> MISC_FEATURE
   <222> (1)..(287)
<400> 26
   Met Thr Gly Phe Gly Ser Pro Cys Gly Ala Cys Lys Phe Leu Arg Arg
   1               5                   10                  15
   Lys Cys Val Arg Gly Cys Val Phe Ala Pro Tyr Phe Cys His Glu Gln
               20                  25                  30
   Gly Ala Ala His Phe Ala Ala Ile His Lys Val Phe Gly Ala Ser Asn
           35                  40                  45
   Val Ser Lys Leu Leu Ala His Leu Pro Leu Ala Asp Arg Pro Glu Ala
       50                  55                  60
   Ala Val Thr Ile Ser Tyr Glu Ala Gln Ala Arg Leu Arg Asp Pro Ile
   65                  70                  75                  80
   Tyr Gly Cys Val Ala His Ile Phe Ala Leu Gln Gln Gln Val Arg Ile
                   85                  90                  95
   Val His Ser Ile Asp Val Ser Leu Val Gly Val Ala Gly Leu Leu Ile
   100 105 110
   Leu Val Ser Arg Arg Val Phe Glu Gln Val Met Thr Leu Gln Ala Gln
   115 120 125
   Leu Ala Ser Leu Lys Ala Ala Ala Ala Gln Gly Ile His His Gln Asp
   130 135 140
   Val Gly Ala Thr Thr Lys Gly Gly Tyr Met Ser Ala Ala Ala Thr Ala
   145 150 155 160
   Ala Asp Asp Gln Leu Gly Tyr Gly Gly Tyr Asn Gln Trp Cys Gly Ser
   165 170 175
   Asn Gly Gly Gly Ala Pro Ala Ala Ser Gln Pro Gly Ala Tyr Ser Ser
   180 185 190
   Asn Gly Gly Ala Gly His Gly His Asp Ser Ile Thr Ala Leu Leu Ala
   195 200 205
   Ala Gly Ser Asp Tyr Met Gln His Ser Leu Tyr His Ala Phe Glu His
   210 215 220
   Ser Glu Gly Ala Gly Ala Val Asp Asp Gly His Ala Ala Ala Ala Ala
   225 230 235 240
   Phe Glu Ala Ala Ala Glu Ser Ser Ser Cys Gly Met Ala Ala Ser Phe
   245 250 255
   Ala Ala Asp Glu Ser Val Trp Arg Ser Ser Ser Ser Gly Tyr Gln Asp
   260 265 270
   Cys Glu Asp Leu Gln Ser Val Ala Tyr Ala Tyr Leu Asn Arg Ser
   275 280 285
<210> 27
   <211> 218
   <212> PRT
   <213> Arabidopsis thaliana
<220>
   <221> MISC_FEATURE
   <222> (1)..(218)
<400> 27
   Met Thr Ser Ser Ser Ser Ser Ser Gly Ser Pro Cys Gly Ala Cys Lys
   1               5                   10                  15
   Phe Leu Arg Arg Lys Cys Ala Lys Gly Cys Val Phe Ala Pro Tyr Phe
               20                  25                  30
   Cys His Glu Gln Gly Ala Ser His Phe Ala Ala Ile His Lys Val Phe
           35                  40                  45
   Gly Ala Ser Asn Ala Ser Lys Leu Leu Ser His Leu Pro Ile Ser Asp
       50                  55                  60
   Arg Cys Glu Ala Ala Ile Thr Ile Ser Tyr Glu Ala Gln Ala Arg Leu
   65                  70                  75                  80
   Gln Asp Pro Ile Tyr Gly Cys Val Ser His Ile Phe Ala Leu Gln Gln
                   85                  90                  95
   Gln Val Val Asn Leu Gln Ala Glu Leu Glu Ile Leu Lys Gln Gln Ala
               100                 105                 110
   Ala Gln Ser Met Ile Phe Ala Asp Ser Pro Thr Ser Glu Asn Pro Asn
           115                 120                 125
   Ser Tyr Tyr Gly Asp Thr Thr Lys Ala Pro Tyr His His Asp His Gln
       130                 135                 140
   Asn Ile Tyr His His His Asp Gln Thr His Leu Val Tyr Gln Thr Gly
   145                 150                 155                 160
   Ser Ser Gly Thr Val Gln His Gly Asp Ala Thr Glu Ser Ser Tyr His
                   165                 170                 175
   Asn Glu Thr Ser Ser Gly Met Gly Glu Phe Ser Ile Tyr Ser Asp Leu
               180                 185                 190
   Glu Gln His Leu Asn Thr Phe Asn Gln Asp His Leu Lys Glu Leu Gln
           195                 200                 205
   Ser Ala Asn Phe Gly Tyr Ile Ser Phe Ser
       210                 215
<210> 28
   <211> 3286
   <212> DNA
   <213> Zea maize
<220>
   <221> gene
   <222> (1)..(3286)
<400> 28
   taggccagac cgccagacta atctggttga gcaaacactt ccagtggcta gtgggaggca      60
   aaaagcctga ttaggatata caactacagg cgccagtacg tactaatcgt ccttttgagc     120
   attctgtgaa gcagaacaga ggcggcagag tttcagaagt tctgctgccc tgttccagtc     180
   cttcataggt gcaaccgtgc aactgctata ctacacgtag gaacagtaca tcttcagcta     240
   tactaaattc agttttttct tcttcttaca aacgcatatt ttaagctaca gcattggcag     300
   gccattgctc gatttgtttt tttctcggtg gttggactta catgcctaca ggaaaactaa     360
   aacaatacgt atatgtggtt ttctgataat caaatcaaag ggggaggggg gatgtgacac     420
   cagaactagt tctttcccat cacccattat tgtttgcttt tgcccagtct cgcgaagaaa     480
   aaaaaatgaa atcaaaagaa aatatcaaag cgacgagcag cgacaactcc acgtctggag     540
   ccaggtgatg tatgagtgca ggtactacac ggtacataga ttttattttt ttaaaaaaaa     600
   atcataagca tttattttat ttatccccaa attatgaact ggactttgct cgctggtctc     660
   gcagcagccg agcccaactg cacacaaaag aaatgggcgc atgagcaggc acagaaaaac     720
   taaacagaga aagcatgcat taattagacc aaacccaaaa cccctaagca aaagattagc     780
   aatgattggc gtctccattg tgcacttgca caggtactag tactcctgct aggcttgttg     840
   ttgtagggtg cctgccccat gcagtgcaag gagggagggg tgtgtcacca taaaaattta     900
   gcggcaaggg cgagggcgat tggaagctca aaataatgag ctggttgccc accggggaga     960
   cacgccggat ttgtttaatc ccctggccct aatcccccag ccctgccgtc tcctccttat    1020
   aagcaatggc ggaggggtct tgctcttgca ttgcacctcc ggccaccgcg ccatagcccg    1080
   cagtaattaa gcaggcgaga acgacgaaga ggcggtcacg caccacaccg atcaatccag    1140
   ctcgagcgac cgatcacacg tgcagcacag cacagcacag cggtgctcgg cgaagagaga    1200
   tgacggggtt cgggtcaccg tgcggggcgt gcaagttcct gcgccgcaag tgcgtgcgcg    1260
   gctgcgtctt cgcgccctac ttctgccacg agcagggcgc ggcgcacttc gccgccatcc    1320
   acaaggtgtt cggcgccagc aacgtgtcca agctgctcgc gcacctgccg ctcgccgacc    1380
   gcgccgaggc cgccgtcacc atctcctacg aggcgcaggc gaggctgcgg gaccccatct    1440
   atggctgcgt cgcccacatc ttcgcgctac agcagcaggt gtgcatgcgc gactgccccg    1500
   ccgcgccgtc tctgggcttg tctcttaatt gtgatagggt ttaattgctg accggcccgt    1560
   gccaatcgat ccaggttatg accctgcagg cgcagctggc gtcgctcaag gcgcaggcgg    1620
   cgcaggggca gcagggcgtg cacgaagacg ccaagggcta cgtgggcagc gccgccgcgg   1680
   agcagctagg gtacggctac ccctggtgca gcggcaatgg aggcgccgca gcagcagcag   1740
   caggcgccgt gggcgcgccc gccgcgcagc cgggcgcgta cggcaatggc gcgcacgagt   1800
   ccctgaccgc gctgctgggg tcgtcggact acatgcagca gtcgctgtac cacgcgttcg   1860
   agcaggccgg cgcggacgac gacgacggcc ggcaggggta cgccttcgag gcagcggcgg   1920
   agtcctcgtc gctcggggcg gaggagagcg ggtggaggtc gtcgtcgggg taccaagact   1980
   gcgaggacct gcagagcgtg gcttacgctt acctgaacca tcgctcgtaa gaactgagaa   2040
   ctactactac tacaagagag agagagagag atatagatat agacatatct gtcctcaatt   2100
   cctgatcatg ttttggactt tagcctgggg aaatatatgc gcgattttcg atcgatcagt   2160
   cgatcggtct ccgctacaaa taatccagaa gcatgcatgc atgtgacaga ccactgatat   2220
   ataatagatc cacacattat tgatcatcag tgtagaaatt aacgtacgta gcctaattaa   2280
   ttggacaaag aaaatggaga gcccttgctg tgattatgct gctagttctg tcagtggtgg   2340
   ggttgtgttt tcttctccaa ctctctgcct acctgctgca gcagtgtctg cagacgataa   2400
   ggttagattc gtcatgccgg ccggaaaatg tactccaagg aacatacaag gcagcatatt   2460
   gagagacagg tgattgattc atggccacac gtggaagatc caattagcct actattttcg   2520
   ttgactcctt ttactggaac tctttctgat gggacatgca cacacatctt cagcatatat   2580
   atagctacta tctagtagat gatatgatag agccttttgt cttgcgtaga caatcctact   2640
   atagtgatta ataactctcc tatatctgga ttattaccct atagctactt gattaccaca   2700
   catgtagata ttctaagtca tgaccattac atccttaaaa aaggataatt atggtgactc   2760
   atcataatta gtgtggtgtc taataatctg tctatttttt cccaggatta catttggtaa   2820
   aaaccttttt tctgaagttt tgtttatttg tacacaaatt ttggttagat gtttccattg   2880
   cgtagaccac catcttttaa accatagatg actgtttcac aaaaaaaagt tgactccttt   2940
   tactggaact ctttctgatg ggacatgcac acatcttcag catatatgta gctactagta   3000
   gatgatatga tagagccttt tgtcttgtgt agacaatcct accatagtga ttaataactc   3060
   tcctatatct ggattattat tgccctatag ctacttgatt aacacacatg tagatattct   3120
   aaatcatgac cattacatac ttaaaaaggg ataattatgg cgactcatca taattagtgt   3180
   ggtgtctaat aattataggt gtacatttgg gccgggtytg atgggccggc ccgaagcacg   3240
   gtaaaaaaag cacggcccaa gcacggcacg gcacgaaata tttagg                  3286
<210> 29
   <211> 735
   <212> DNA
   <213> Zea Maize
<220>
   <221> CDS
   <222> (1)..(735)
<400> 29
   atg acg ggg ttc ggg tca ccg tgc ggg gcg tgc aag ttc ctg cgc cgc      48
   Met Thr Gly Phe Gly Ser Pro Cys Gly Ala Cys Lys Phe Leu Arg Arg
   1               5                   10                  15
   aag tgc gtg cgc ggc tgc gtc ttc gcg ccc tac ttc tgc cac gag cag      96
   Lys Cys Val Arg Gly Cys Val Phe Ala Pro Tyr Phe Cys His Glu Gln
               20                  25                  30
   ggc gcg gcg cac ttc gcc gcc atc cac aag gtg ttc ggc gcc agc aac     144
   Gly Ala Ala His Phe Ala Ala Ile His Lys Val Phe Gly Ala Ser Asn
           35                  40                  45
   gtg tcc aag ctg ctc gcg cac ctg ccg ctc gcc gac cgc gcc gag gcc     192
   Val Ser Lys Leu Leu Ala His Leu Pro Leu Ala Asp Arg Ala Glu Ala
       50                  55                  60
   gcc gtc acc atc tcc tac gag gcg cag gcg agg ctg cgg gac ccc atc     240
   Ala Val Thr Ile Ser Tyr Glu Ala Gln Ala Arg Leu Arg Asp Pro Ile
   65                  70                  75                  80
   tat ggc tgc gtc gcc cac atc ttc gcg cta cag cag cag gtg atg acc     288
   Tyr Gly Cys Val Ala His Ile Phe Ala Leu Gln Gln Gln Val Met Thr
                   85                  90                  95
   ctg cag gcg cag ctg gcg tcg ctc aag gcg cag gcg gcg cag ggg cag     336
   Leu Gln Ala Gln Leu Ala Ser Leu Lys Ala Gln Ala Ala Gln Gly Gln
               100                 105                 110
   cag ggc gtg cac gaa gac gcc aag ggc tac gtg ggc agc gcc gcc gcg     384
   Gln Gly Val His Glu Asp Ala Lys Gly Tyr Val Gly Ser Ala Ala Ala
           115                 120                 125
   gag cag cta ggg tac ggc tac ccc tgg tgc agc ggc aat gga ggc gcc     432
   Glu Gln Leu Gly Tyr Gly Tyr Pro Trp Cys Ser Gly Asn Gly Gly Ala
       130                 135                 140
   gca gca gca gca gca ggc gcc gtg ggc gcg ccc gcc gcg cag ccg ggc     480
   Ala Ala Ala Ala Ala Gly Ala Val Gly Ala Pro Ala Ala Gln Pro Gly
   145                 150                 155                 160
   gcg tac ggc aat ggc gcg cac gag tcc ctg acc gcg ctg ctg ggg tcg     528
   Ala Tyr Gly Asn Gly Ala His Glu Ser Leu Thr Ala Leu Leu Gly Ser
                   165                 170                 175
   tcg gac tac atg cag cag tcg ctg tac cac gcg ttc gag cag gcc ggc     576
   Ser Asp Tyr Met Gln Gln Ser Leu Tyr His Ala Phe Glu Gln Ala Gly
               180                 185                 190
   gcg gac gac gac gac ggc cgg cag ggg tac gcc ttc gag gca gcg gcg     624
   Ala Asp Asp Asp Asp Gly Arg Gln Gly Tyr Ala Phe Glu Ala Ala Ala
           195                 200                 205
   gag tcc tcg tcg ctc ggg gcg gag gag agc ggg tgg agg tcg tcg tcg       672
   Glu Ser Ser Ser Leu Gly Ala Glu Glu Ser Gly Trp Arg Ser Ser Ser
       210                 215                 220
   ggg tac caa gac tgc gag gac ctg cag agc gtg gct tac gct tac ctg       720
   Gly Tyr Gln Asp Cys Glu Asp Leu Gln Ser Val Ala Tyr Ala Tyr Leu
   225                 230                 235                 240
   aac cat cgc tcg taa                                                   735
   Asn His Arg Ser
<210> 30
   <211> 244
   <212> PRT
   <213> Zea Maize
<400> 30
   Met Thr Gly Phe Gly Ser Pro Cys Gly Ala Cys Lys Phe Leu Arg Arg
   1               5                   10                  15
   Lys Cys Val Arg Gly Cys Val Phe Ala Pro Tyr Phe Cys His Glu Gln
               20                  25                  30
   Gly Ala Ala His Phe Ala Ala Ile His Lys Val Phe Gly Ala Ser Asn
           35                  40                  45
   Val Ser Lys Leu Leu Ala His Leu Pro Leu Ala Asp Arg Ala Glu Ala
       50                  55                  60
   Ala Val Thr Ile Ser Tyr Glu Ala Gln Ala Arg Leu Arg Asp Pro Ile
   65                  70                  75                  80
   Tyr Gly Cys Val Ala His Ile Phe Ala Leu Gln Gln Gln Val Met Thr
                   85                  90                  95
   Leu Gln Ala Gln Leu Ala Ser Leu Lys Ala Gln Ala Ala Gln Gly Gln
               100                 105                 110
   Gln Gly Val His Glu Asp Ala Lys Gly Tyr Val Gly Ser Ala Ala Ala
           115                 120                 125
   Glu Gln Leu Gly Tyr Gly Tyr Pro Trp Cys Ser Gly Asn Gly Gly Ala
       130                 135                 140
   Ala Ala Ala Ala Ala Gly Ala Val Gly Ala Pro Ala Ala Gln Pro Gly
   145                 150                 155                 160
   Ala Tyr Gly Asn Gly Ala His Glu Ser Leu Thr Ala Leu Leu Gly Ser
   165 170 175
   Ser Asp Tyr Met Gln Gln Ser Leu Tyr His Ala Phe Glu Gln Ala Gly
   180 185 190
   Ala Asp Asp Asp Asp Gly Arg Gln Gly Tyr Ala Phe Glu Ala Ala Ala
   195 200 205
   Glu Ser Ser Ser Leu Gly Ala Glu Glu Ser Gly Trp Arg Ser Ser Ser
   210 215 220
   Gly Tyr Gln Asp Cys Glu Asp Leu Gln Ser Val Ala Tyr Ala Tyr Leu
   225 230 235 240
   Asn His Arg Ser
<210> 31
   <211> 23
   <212> DNA
   <213> primer
<400> 31
   gtgatgaccc tgcaggcgca gct 23
<210> 32
   <211> 25
   <212> DNA
   <213> primer
<400> 32
   gcagtcttgg taccccgacg acgac 25
<210> 33
   <211> 24
   <212> DNA
   <213> primer
<400> 33
   gaagaagcag cataagcaga agca 24
<210> 34
   <211> 22
   <212> DNA
   <213> primer
<400> 34
   cgacgctctg gaggtcctcg ca 22
<210> 35
   <211> 24
   <212> DNA
   <213> primer
<400> 35
   atgacggggt tcgggtcacc gtgc 24
<210> 36
   <211> 30
   <212> DNA
   <213> primer
<400> 36
   ttacgagcga tggttcaggt aagcgtaagc 30
<210> 37
   <211> 699
   <212> DNA
   <213> Zea mays
<400> 37
   atgacggggt tcgggtcacc gtgcggagcg tgcaagttcc tgcggcgcag gtgcgcgcgc     60
   ggctgcgtct tcgcgcccta cttctgcgac gagcagggcg cggcgcactt cgccgccatc    120
   cacaaggtgt tcggcgccag caacgcgtcc aagctgctcg cgcacctgcc gctcgccgac    180
   cgcccggagg ccgccgccac catctcctac gaggcgcagg cgaggctgcg cgaccccacc    240
   tacggctgcg tcgcccacat cttcgcgctc cagcagcagg tgatggccct gcagacgcag    300
   ctggcgtcgc tcacggtcac ggcgcagggg cagcagcgcg tgcacgacgt cgacgacgcc    360
   aagggctacg tgggcaccgc cgccgcgggg cagctagggt gcagcagcaa tggaagcgtc    420
   gcgccggcca cgtacggcaa tgtcgggcac gagtccctga ccgcgctgct gaggtcggag    480
   tcggactact tgcagcagtc gccgtgccac gcgttcgagc acgccggcgc ggacgacaag    540
   gacgacgacg gccggcaggg gaacaccttc gacttcgagg cagcggccga ctcctcgtcg    600
   ttcggggcgg aggagagcgg gtggaggtcg ttgtcagggt accgagactg cgaggacctg    660
   cagagcgtgg cttacgctta cctgaaccat cgctcgtaa                           699
<210> 38
   <211> 232
   <212> PRT
   <213> Zea mays
<400> 38
   Met Thr Gly Phe Gly Ser Pro Cys Gly Ala Cys Lys Phe Leu Arg Arg
   1 5 10 15
   Arg Cys Ala Arg Gly Cys Val Phe Ala Pro Tyr Phe Cys Asp Glu Gln
   20 25 30
   Gly Ala Ala His Phe Ala Ala Ile His Lys Val Phe Gly Ala Ser Asn
   35 40 45
   Ala Ser Lys Leu Leu Ala His Leu Pro Leu Ala Asp Arg Pro Glu Ala
   50 55 60
   Ala Ala Thr Ile Ser Tyr Glu Ala Gln Ala Arg Leu Arg Asp Pro Thr
   65 70 75 80
   Tyr Gly Cys Val Ala His Ile Phe Ala Leu Gln Gln Gln Val Met Ala
   85 90 95
   Leu Gln Thr Gln Leu Ala Ser Leu Thr Val Thr Ala Gln Gly Gln Gln
   100 105 110
   Arg Val His Asp Val Asp Asp Ala Lys Gly Tyr Val Gly Thr Ala Ala
   115 120 125
   Ala Gly Gln Leu Gly Cys Ser Ser Asn Gly Ser Val Ala Pro Ala Thr
   130 135 140
   Tyr Gly Asn Val Gly His Glu Ser Leu Thr Ala Leu Leu Arg Ser Glu
   145 150 155 160
   Ser Asp Tyr Leu Gln Gln Ser Pro Cys His Ala Phe Glu His Ala Gly
   165 170 175
   Ala Asp Asp Lys Asp Asp Asp Gly Arg Gln Gly Asn Thr Phe Asp Phe
   180 185 190
   Glu Ala Ala Ala Asp Ser Ser Ser Phe Gly Ala Glu Glu Ser Gly Trp
   195 200 205
   Arg Ser Leu Ser Gly Tyr Arg Asp Cys Glu Asp Leu Gln Ser Val Ala
   210 215 220
   Tyr Ala Tyr Leu Asn His Arg Ser
   225 230
<210> 39
<211> 5276
   <212> DNA
   <213> Zea mays
<400> 39
   ccttgtctag taaacattgt catgcaaaaa ccattgctgt gtagtcatgt gctgttttat 60
   ttcccgttgg gttgaaaatg tcagccatag ttttcagatt gccttgtgtt gctctattta 120
   gcgcccattg tctgctaagc atcgtcactt tcctgcggcc acattgctaa tagtatgcta 180
   tatggagtca cttatgggcc tccggtgaca gacatctatc tactgttctt tttttattgt 240
   gaatctcgac agatacgatc ttttggtttg tctccgtgtc gctacccgag ctactagcta 300
   atgaagtcac ttcattttcg cagctgctgt acctcgctct gatcactcct atcgttcacg 360
   acttctacaa ctacgacacg gagaaggaag aattcgcgcg gctgtttgcg aagttcaccc 420
   aggtaactat ggggttcgcc gttcagaaat ggttccatgc catggccatg ccatgctgtt 480
   gtggtagtat ctgatcagcc gacgctaact tggcaggacg tggcgctggt tggagctctc 540
   ctcgtcttcc tgggcatgaa gaactccatc ccgaagcggc agggcaagaa gaaggctcac 600
   aaggcgaaga cgaactagta ggaggctgct cgctctttct ttagatggtt gcgcgccggc 660
   ctcaggcggg tgtcaggaga cgaataggcc cactagtgcg aagcagtcgt cgatgcgcat 720
   cgagctgccg gctgcgcttt tacatgtatc ggatattcag attccatttt gcggggcgcg 780
   gcccgaacga aggtgtgact agtgagcggc ccgaacgaag gtgtgactag tgagcggcgg 840
   tcatgtatgt gattgcgatt ggttggttgg tttcttgaac tgggctggca gtggttaatc 900
   ttttcgattt gattatcttt tgcactgggc cgcaactgat tcgatcagga aaagtcatca 960
   ctgtccgaaa aacttacttg tagaggtagg gcatgtttgg ttggatgccc aaaaattgct 1020
   acacattttc tgccatgttt gcccaaagga atcttgccat atttttaaca gttattgaca 1080
   agcaacgaac caaacagcct cgtaactcat ctgaaattaa atttgtagct gcggctattt 1140
   ttcctagttg cctctacgaa tccagttact tgacacctgc ctctagaaat tgctgattta 1200
   tggagagaaa agactagaga cagccacctt tatagaggca tcctaaccgc ctctaaagac 1260
   aaaaactata gtagtggcta tatatatgtt tgacgtaaac caagctatat gtgtctattt 1320
   aagtgagaat ttattctcgc gtctcccttc cacacttaag gctcaaaacc ttaaccctct 1380
   atctcctaga cttagctcct aaccactgaa ttgcatattt gtttatgttc tatagaagat 1440
   agatctctac atatttaagt gggttcatgt cccccttcgt gcgtaccacc agaaaaatac 1500
   aaaaaaagcg tgaggttcga accctaacct tctatcttct agactcattt tccctatttt 1560
   ctaaccacta ggatacatat ttatttgggt tttaaaaaag gtagaaatta tagatataga 1620
   aactgtagta agacacgagc aacctacagg tatttatata ccgcgtatat tcactagtat 1680
   ccaaaacaac ttaactacgg acgatgctgg cgggctttat atatgtgtgt gaagcaaacc   1740
   aaaactagtg cagtagctag acagtgttgt agcatctgaa agcacacaca attaacataa   1800
   cccatacacg gaaacacaag gcttagtcag ctattgctat ttggtacgag tttttccatg   1860
   tgtgtgcctg ccgggagggt gaatggatga gacgagagca ccacgccacc acctctagtt   1920
   gtttgttgac tgcactccac gggacttgtg gaacagaaca gacaacaaga cccaccactg   1980
   accacggccc atcggctcac ctcccaggaa tacttggaac atgtccattc cctgctcctc   2040
   atctgcactg cacgccatgc caaacctctt ttccatccgg cggcttgcat agttttttta   2100
   gttccacttc ccttgcagat aagtgaggag aaagccgccg tttttcatat gtagcctcgc   2160
   gagatgtgtt gaaagaaaag aaagaaagaa aaaaagccag tagcagcagt agcacgacag   2220
   gataagctga agtgacagga agatgagcat agtttggccg ctgttcagag cgatcgtgct   2280
   cgatgcgatc ttccattcag ttcccgctcc aaattcagta caactatcta gtgcagattg   2340
   taagtacaga atccgttgta ttttgctttc tgactacaga aggatcatgt cgtgcgctac   2400
   aaaagtctgc tagctgcaga acaacgagat ggaataatac tcctagagtc ctagttgcta   2460
   cttactacag tacctcacaa gttttttctt cttcttcttg aaagaccacc tcgcaagctt   2520
   ttcgaatgtg aacaaatagc aataaaacaa gtcagagttg tctctgatcc agtttccaat   2580
   gctcgacaga ttaacgctag ctgttcccat aggatgaaca agcctgcagg attaaagaaa   2640
   caatgagaga aagagctgcg ttgtggtcgt cgtggggcca acatatacac acatgcaccg   2700
   ctgggtacta cgtaactagg ggggtggggt ggctccgtac tacaaaaaga tggctcgaat   2760
   cgactcgtta caataagagt tagaatactt gctcaattcg gttcgttaga caacttaagt   2820
   cagctcgact gactcgtaag ctagaccaaa attacacccc ggtaacattg caaatcaatc   2880
   tatcattttc tttgtccatc agattttgac gtataaaatt tcactacgca ttgcacagtt   2940
   acattaaggc tagcaacagg caaatacaag tcgacatttg aaaaatacta tgcaactatg   3000
   gtccttgaac gcgtcttagc ggttgccgaa ggtgtgttca tcacctgatg acgctgacaa   3060
   gtttaaatct gaaacacaag cacaatggca atttagattg agggccctta attaaattta   3120
   attcaaaaat aaataagaat agagtctggt cctaattcga tttggcattt aaatgttatt   3180
   gtgtaaaatt tagaactcat taccatcctc agtggctagt ggaaagcaaa aggcctgatt   3240
   acgataaaac tacatgcgtc agtactaacc gtccttttgg gcgttttgtg aagcagaggc   3300
   agcagagttt cagaaattct gctgccatgt tcctcagtcc ttcaaaggtg caactgctat   3360
   atactataga aagaagaaaa cagtacatct taagctataa caaattcagt tctattataa   3420
   acacataatt taagctacga attggcagac cattgcttga tttgtttttc tcggtggctg   3480
   gacttacatg cctacataaa aaaacaaaag gaatatatat atatagttta ctgataatca 3540
   aaccaaaggg gagatgctga cacaagaact agttctttcc tatcactgga taccatgtgc 3600
   taaactttat ggtgctaaag tttaacatac cttgaggagt taaactgtta ttggatgtta 3660
   aagtttttca cttcactttt aacattcatg tttggatctt aagtactaaa agtataatgc 3720
   taaactttag catacttaag aaactaaact ggtattgggt actaaagtgc taaaaggtca 3780
   taaaatgaaa tgctagactt tagcacagtg tatcaaatag gtccttagtc tcccaaaaca 3840
   aaaagagaga gagagagaga taaggaaata gtactcctgc taggcttatt gtagtagggt 3900
   gccccatgcg gtgcagggag ggtgtcacca aaaaaaaact tagcggtaag gagcgagggc 3960
   gattggaagc tcaaaataat ggagctggtt gtccaccggg cagacacgcc tggtttgttt 4020
   aatcctttgg ccctaatccc ccagccctgc tgccgtctcc ttctcctcct tataagcatt 4080
   gccgccccgc ccccatcctc caccgcacca cagccgcagt cagcagggca agaacacgaa 4140
   gtgatcgtcg ccaccgatcg atccagctcg agctgagcga gctatcacgt gcagcacagt 4200
   gcagtacaga acagctgtgc ttgcagtctt gcagatggag atgacggggt tcgggtcacc 4260
   gtgcggagcg tgcaagttcc tgcggcgcag gtgcgcgcgc ggctgcgtct tcgcgcccta 4320
   cttctgcgac gagcagggcg cggcgcactt cgccgccatc cacaaggtgt tcggcgccag 4380
   caacgcgtcc aagctgctcg cgcacctgcc gctcgccgac cgcccggagg ccgccgccac 4440
   catctcctac gaggcgcagg cgaggctgcg cgaccccacc tacggctgcg tcgcccacat 4500
   cttcgcgctc cagcagcagg tgcactacgg ctgcgtgcac gaagtgcctg gcggctgccg 4560
   ccgccgccgt ctccgctttg tctctcgtga cggggtttaa tttgctgatg acccgtgccg 4620
   ccgtgcaccc cgattccgat ggatccaggt gatggccctg cagacgcagc tggcgtcgct 4680
   cacggtcacg gcgcaggggc agcagcgcgt gcacgacgtc gacgacgcca agggctacgt 4740
   gggcaccgcc gccgcggggc agctagggtg cagcagcaat ggaagcgtcg cgccggccac 4800
   gtacggcaat gtcgggcacg agtccctgac cgcgctgctg aggtcggagt cggactactt 4860
   gcagcagtcg ccgtgccacg cgttcgagca cgccggcgcg gacgacaagg acgacgacgg 4920
   ccggcagggg aacaccttcg acttcgaggc agcggccgac tcctcgtcgt tcggggcgga 4980
   ggagagcggg tggaggtcgt tgtcagggta ccgagactgc gaggacctgc agagcgtggc 5040
   ttatgcttac ctgaaccatc tctcttaaaa gagctgagaa ctacttactc gtagtgccgg 5100
   aaatgtatca caagagatca gagagagaga tcatgttttg gacttgagct tgggatgtgt 5160
   gcgattttcg atcggtctcc gctagaaaca attcagaagg tagcatgcat gtgacagccc 5220
   gttgatatac tctcgccgtt cttttattta tttatttatt tatttattta tttatt 5276
<210> 40
   <211> 21
   <212> DNA
   <213> primer
<400> 40
   ggcaggccat tgctcgattt g 21
<210> 41
   <211> 22
   <212> DNA
   <213> primer
<400> 41
   gcacttgcgg cgcaggaact tg 22
<210> 42
   <211> 3864
   <212> DNA
   <213> Zea mays
<400> 42

## Description

### FIELD OF THE INVENTION

The field of invention relates to plant breeding and genetics and, in particular, relates to recombinant constructs useful for altering root structure during plant development.

### BACKGROUND OF THE INVENTION

Relatively little is known about the genetic regulation of plant root development and function. Elucidation of the genetic regulation is important because roots serve important functions such as acquisition of water and nutrients and the anchorage of the plants in the soil.

The mutation of the *RTCS* (rootless for crown and seminal roots) gene was first described by Hetz et al. (1996) Plant J.10(5):845-857. Two maize *rtcs* mutants, rtcs1 and rtcs2, were shown to have reduced resistance to root lodging. Both mutants were found to be deficient in formation of both crown and seminal lateral roots, which appear to be suppressed at a very early stage in mutant embryos. In addition, brace root formation, which occurs at a later stage of development in wild type-plants, was also found to be lacking in the two rtcs mutants. Microscopic analysis further showed that root primordia formation was absent in mutant plants.

The mutation was genetically mapped to the short arm of chromosome 1, with apparently no phenotypic differences between the two mutants. Genetic analysis of the two rtcs mutations indicate that they are both inherited as monogenic recessive traits. More extended mapping analysis and a narrowing of the location of the mutant locus on chromosome 1 was performed as described in Krebs et al, (1999) MNL 73:33.

Despite the extensive genetic and morphological characterization of the *rtcs* mutants, there has been no molecular analysis of the nucleic acid encoding the protein associated with the rtcs phenotype. Indeed, the identity of the protein encoded by *RTCS* has not been reported. A hypothetical protein sequence of 287 amino acids from rice is disclosed in the NCBI Database at Accession No. AAN87738.1 (GI No. 27261472).

It has been reported that the Lateral Organ Boundary (LOB) gene in Arabidopsis has a potential role in lateral organ development. See Shuai et al., (2002), Plant Phys. 129, 747-761. Shuai et al. found LOB gene expression at the base of lateral organs in the shoots and roots of Arabidopsis. Morever, 23 members of the LOB domain family (LBD) of genes were found to exhibit expression patterns in the root tissues of Arabidopsis.

### SUMMARY OF THE INVENTION

The present invention includes isolated polynucleotides encoding a polypeptide required for proper root formation, wherein the absence of or altered levels of the polypeptide in a plant results in the elimination of or altered levels of formation of one or more of the nodal roots, wherein the amino acid sequence of the polypeptide and the amino acid sequence of SEQ ID NO: 6 , 8, 30, or 38 have at least 70%, 75%, 80%, 85%, 90%, 95%, 99% or 100% identity, or the complement of the nucleotide sequence, wherein the complement and the nucleotide sequence contain the same number of nucleotides and are 100% complementary. The polypeptide preferably comprises the amino acid sequence of SEQ ID NO:6, 8, 30, or 38. The nucleotide sequence preferably comprises the nucleotide sequence of SEQ ID NO: 5, 7, 29, or 37.

In a first embodiment, the present invention includes an isolated polynucleotide comprising: (a) a nucleotide sequence encoding a polypeptide required for proper root formation, wherein the absence of or altered levels of the polypeptide in a plant results in the elimination of or altered levels of formation of one or more of the nodal roots, wherein the polypeptide has an amino acid sequence of at least 70%, 75%, 80%, 85%, 90%, 95%, 99% or 100% sequence identity based on the Clustal V method of alignment when compared to a polypeptide SEQ ID NO:6, 8, 30, or 38, or (b) a complement of the nucleotide sequence, wherein the complement and the nucleotide sequence contain the same number of nucleotides and are 100% complementary.

In a second embodiment, this invention includes isolated polynucleotide sequences comprising a sequence which encodes at least two motifs selected from the group consisting of SEQ ID NOs:9,10,11,12 and 13, wherein said motif is substantially a conserved subsequence.

In a third embodiment, this invention includes a functionally equivalent subfragment of an isolated polynucleotide (or complement) of the present invention, wherein the subfragment is useful in antisense inhibition or co-suppression of a nucleic acid sequence encoding a polypeptide as defined in the first embodiment.

In a fourth embodiment, this invention includes an isolated nucleic acid fragment comprising a promoter wherein said promoter consists essentially of the nucleotide sequence set forth in SEQ ID NO:2,3 or 4 or a substantially similar and functionally equivalent subfragment of said promoter.

In a fifth embodiment, this invention includes recombinant DNA constructs comprising any of the foregoing nucleic acid fragments or complements or functionally equivalent subfragments, operably linked to at least one regulatory sequence. Also included are plants comprising such recombinant DNA constructs in their genome, plant tissue or cells obtained from such plants, and seeds obtained from these plants.

In a sixth embodiment, this invention includes a method of altering root structure during plant development in plants which comprises:
(a) transforming a plant with a recombinant DNA construct of the invention;
(b) growing the transformed plant under conditions suitable for the expression of the recombinant DNA construct; and
(c) selecting those transformed plants having altered root structure.

In a seventh embodiment, this invention includes a method to isolate nucleic acid fragments encoding polypeptides associated with altering root structure during plant development which comprises:
(a) comparing SEQ ID NO's: 6, 8, 30,or 38 with other polypeptide sequences associated with altering root structureduring plant development;
(b) identifying the conserved sequences(s) of 4 or more amino acids obtained in step (a);
(c) making region-specific nucleotide probe(s) or oligomer(s) based on the conserved sequences identified in step (b); and
(d) using the nucleotide probe(s) or oligomer(s) of step (c) to isolate sequences associated with altering root structure during plant development by sequence dependent protocols.

Also disclosed is a method of mapping genetic variations related to altering root structure during plant development comprising:
(a) crossing two plant varieties; and
(b) evaluating genetic variations with respect to:
   (i) a nucleic acid sequence selected from the group consisting of SEQ ID NO: 5, 7 , 29, or 37; or
   (ii) a nucleic acid sequence encoding a polypeptide selected from the group consisting of SEQ ID NO: 6, 8, 30, or 38; in progeny plants resulting from the cross of step (a), wherein the evaluation is made using a method selected from the group consisting of: RFLP analysis, SNP analysis, and PCR-based analysis.

Also disclosed is a method of molecular breeding to alter root structure during plant development, the method comprising:
(a) crossing two plant varieties; and
(b) evaluating genetic variations with respect to:
   (i) a nucleic acid sequence selected from the group consisting of SEQ ID NO:5 ,7,29, or 37; or
   (ii) a nucleic acid sequence encoding a polypeptide selected from the group consisting of SEQ ID NO: 6, 8 ,30 or 38
      in progeny plants resulting from the cross of step (a), wherein the evaluation is made using a method selected from the group consisting of: RFLP analysis, SNP analysis, and PCR-based analysis.

### BRIEF DESCRIPTION OF THE FIGURES AND SEQUENCE LISTINGS

The invention can be more fully understood from the following detailed description and the accompanying drawings and Sequence Listing, which form a part of this application.
Figure 1 shows an alignment of the RTCS polypeptide sequence from maize inbred line Mo17 (SEQ ID NO:6), the deduced rice polypeptide sequence (SEQ ID NO:8), the RTCS polypeptide sequence from maize inbred line B73 (SEQ ID NO:30), a hypothetical protein sequence from rice (SEQ ID NO: 26, GI No. 27261472), and a putative LOB domain protein sequence from *Arabidopsis* (SEQ ID NO: 27, GI No. 22331847).
Figure 2 shows vector PHP24451.
Figure 3 shows vector PHP24452.
Figure 4 shows the rtcs allele described in Example 10.
SEQ ID NO:1 represents the 6101 bp of the maize genomic sequence containing the ORF (Nucleotides 2779-3610) of the RTCS gene, which is interrupted by an intron extending from Nucleotides 3060-3159.
SEQ ID NO:2 is 2778 bp, extending from Nucleotides 1-2778 of the putative promoter of the maize RTCS genomic sequence shown in SEQ ID NO:1.
SEQ ID NO:3 is 2000 bp, extending from Nucleotides 779 -2778 of the putative promoter of the maize RTCS genomic sequence shown in SEQ ID NO:1.
SEQ ID NO:4 is 1000 bp, extending from Nucleotide 1779-2778 of the putative promoter of the maize RTCS genomic sequence shown in SEQ ID NO:1.
SEQ ID NO:5 is the nucleotide sequence of the ORF of SEQ ID NO:1 minus an intron sequence (i.e., SEQ ID NO:5 is nucleotides 2779-3059 and 3160-3610 of SEQ ID NO:1).
SEQ ID NO:6 is the amino acid sequence encoded by SEQ ID NO:5.
SEQ ID NO:7 is the deduced nucleotide sequence for the coding sequence of the rice RTCS gene.
SEQ ID NO:8 is the deduced amino acid sequence encoded by SEQ ID NO:7.
SEQ ID NO:9 is a conserved sequence motif associated with nucleotide sequences included in the present invention.
SEQ ID NO:10 is a conserved sequence motif associated with nucleotide sequences included in the present invention.
SEQ ID NO:11 is a conserved sequence motif associated with nucleotide sequences included in the present invention.
SEQ ID NO:12 is a conserved sequence motif associated with nucleotide sequences included in the present invention.
SEQ ID NO:13 is a conserved sequence motif associated with nucleotide sequences included in the present invention.
SEQ ID NO:14 is the forward primer for SSR marker BNLG1014 used in Example 1.
SEQ ID NO:15 is the reverse primer for SSR marker BNLG 1014 used in Example 1.
SEQ ID NO:16 is the forward primer for SSR marker BNLG 1429 used in Example 1.
SEQ ID NO:17 is the reverse primer for SSR marker BNLG 1429 used in Example 1.
SEQ ID NO:18 is the forward primer for SSR marker UMC1685 used in Example 1.
SEQ ID NO:19 is the reverse primer for SSR marker UMC 1685 used in Example 1.
SEQ ID NO:20 is the forward primer for SSR marker UMC1660 used in Example 1.
SEQ ID NO:21 is the reverse primer for SSR marker UMC 1660 used in Example 1.
SEQ ID NO:22 is the forward primer for Cap marker b104.i24 used in Example 1.
SEQ ID NO:23 is the reverse primer for Cap marker b104.i24 used in Example 1.
SEQ ID NO:24 is the forward primer of Cap marker b74.m9 used in Example 1.
SEQ ID NO:25 is the reverse primer of Cap marker b74.m9 used in Example 1.
SEQ ID NO:26 shows the amino acid sequence for the rice hypothetical protein gi: 27261472.
SEQ ID NO:27 shows the amino acid sequence for the Arabidopsis putative LOB domain protein gi: 22331847.
SEQ ID NO:28 represents the 3286 bp of the maize genomic sequence containing the ORF (Nucleotides 1200 - 2030) of the RTCS gene, which is interrupted by an intron extending from Nucleotides 1482-1577.
SEQ ID NO:29 is the nucleotide sequence of the ORF of SEQ ID NO:28 minus an intron sequence (i.e., SEQ ID NO:29 is nucleotides 1200-1481 and 1578-2030 of SEQ ID NO:28).
SEQ ID NO:30 is the amino acid sequence encoded by SEQ ID NO:29.
SEQ ID NO:31 is the forward primer used in Example 4.
SEQ ID NO:32 is the reverse primer used in Example 4.
SEQ ID NO:33 is the forward primer used in Example 6.
SEQ ID NO:34 is the reverse primer used in Example 6.
SEQ ID NO:35 is the forward primer used in Example 9.
SEQ ID NO:36 is the reverse primer used in Example 9.
SEQ ID NO:37 is the RTCS-like cDNA obtained from the experiment described in Example 9.
SEQ ID NO:38 is the amino acid sequence encoded by SEQ ID NO: 37. SEQ ID NO:39 is the genomic sequence containing the RTCS-like gene. SEQ ID NO:40 is the forward primer used in Example 10.
SEQ ID NO:41 is the reverse primer used in Example 10.
SEQ ID NO:42 is the 3864 bp nucleotide sequence containing the 1536bp of the rtcs mutant gene described in Example 10.

The sequence descriptions and Sequence Listing attached hereto comply with the rules governing nucleotide and/or amino acid sequence disclosures in patent applications as set forth in 37 C.F.R. §1.821-1.825.

The Sequence Listing contains the one letter code for nucleotide sequence characters and the three letter codes for amino acids as defined in conformity with the IUPAC-IUBMB standards described in *Nucleic Acids Res. 13*:3021-3030 (1985) and in the *Biochemical J. 219 (No. 2)*:345-373 (1984). The symbols and format used for nucleotide and amino acid sequence data comply with the rules set forth in 37 C.F.R. §1.822.

### DETAILED DESCRIPTION OF THE INVENTION

As used herein, an "isolated nucleic acid fragment" is used interchangeably with "isolated polynucleotide" and is a polymer of RNA or DNA that is single- or double-stranded, optionally containing synthetic, non-natural or altered nucleotide bases. An isolated nucleic acid fragment in the form of a polymer of DNA may be comprised of one or more segments of cDNA, genomic DNA or synthetic DNA. Nucleotides (usually found in their 5'-monophosphate form) are referred to by their single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide.

The term "isolated" refers to materials, such as nucleic acid molecules and/or proteins, which are substantially free or otherwise removed from components that normally accompany or interact with the materials in a naturally occurring environment. Isolated polynucleotides may be purified from a host cell in which they naturally occur. Conventional nucleic acid purification methods known to skilled artisans may be used to obtain isolated polynucleotides. The term also embraces recombinant polynucleotides and chemically synthesized polynucleotides.

The terms "subfragment that is functionally equivalent" and "functionally equivalent subfragment" are used interchangeably herein. These terms refer to a portion or subsequence of an isolated nucleic acid fragment in which the ability to alter gene expression or produce a certain phenotype is retained whether or not the portion or subsequence encodes an active enzyme or functional protein (for example, the portion or subsequence may be a portion of coding and/or non-coding regions and need not encode an active enzyme or functional protein. For example, the fragment or subfragment can be used in the design of recombinant DNA constructs to produce the desired phenotype in a transformed plant. Recombinant DNA constructs can be designed for use in co-suppression or antisense by linking a nucleic add fragment or subfragment thereof, whether or not it encodes an active enzyme or functional protein, in the appropriate orientation relative to a plant promoter sequence.

The terms "homology", "homologous", "substantially similar" and " corresponding substantially" are used interchangeably herein. They refer to nucleic acid fragments wherein changes in one or more nucleotide bases does not affect the ability of the nucleic acid fragment to mediate gene expression or produce a certain phenotype. These terms also refer to modifications of the nucleic acid fragments of the instant invention such as deletion or insertion of one or more nucleotides that do not substantially alter the functional properties of the resulting nucleic acid fragment relative to the initial, unmodified fragment. It is therefore understood, as those skilled in the art will appreciate, that the invention encompasses more than the specific exemplary sequences.

Moreover, the skilled artisan recognizes that substantially similar nucleic acid sequences may also be defined by their ability to hybridize, under moderately stringent conditions (for example, 1 X SSC, 0.1 % SDS, 60°C) with the sequences exemplified herein, or to any portion of the nucleotide sequences reported herein. Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions. One set of preferred conditions involves a series of washes starting with 6X SSC, 0.5% SDS at room temperature for 15 min, then repeated with 2X SSC, 0.5% SDS at 45°C for 30 min, and then repeated twice with 0.2X SSC, 0.5% SDS at 50°C for 30 min. A more preferred set of stringent conditions involves the use of higher temperatures in which the washes are identical to those above except for the temperature of the final two 30 min washes in 0.2X SSC, 0.5% SDS was increased to 60°C. Another preferred set of highly stringent conditions involves the use of two final washes in 0.1X SSC, 0.1% SDS at 65°C.

With respect to the degree of substantial similarity between the target (endogenous) mRNA and the RNA region in the construct having homology to the target mRNA, such sequences should be at least 25 nucleotides in length, preferably at least 50 nucleotides in length, more preferably at least 100 nucleotides in length, again more preferably at least 200 nucleotides in length, and most preferably at least 300 nucleotides in length; and should be at least 80% identical, preferably at least 85% identical, more preferably at least 90% identical, and most preferably at least 95% identical.

Sequence alignments and percent similarity calculations may be determined using a variety of comparison methods designed to detect homologous sequences including, but not limited to, the Megalign program of the LASARGENE bioinformatics computing suite (DNASTAR Inc., Madison, WI). Multiple alignment of the sequences are performed using the Clustal method of alignment (Higgins and Sharp (1989) CABIOS. 5:151-153) with the default parameters (GAP PENALTY=10, GAP LENGTH PENALTY=10). Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4.

"Gene" refers to a nucleic acid fragment that expresses a specific protein, including regulatory sequences preceding (5' non-coding sequences) and following (3' non-coding sequences) the coding sequence. "Native gene" refers to a gene as found in nature with its own regulatory sequences. "Recombinant DNA construct" refers to a combination of nucleic acid fragments that are not normally found together in nature. Accordingly, a recombinant DNA construct may comprise regulatory sequences and coding sequences that are derived from different sources, or regulatory sequences and coding sequences derived from the same source, but arranged in a manner different than that normally found in nature. A "foreign" gene refers to a gene not normally found in the host organism, but that is introduced into the host organism by gene transfer. Foreign genes can comprise native genes inserted into a non-native organism, or recombinant DNA constructs. A "transgene" is a gene that has been introduced into the genome by a transformation procedure.

"Coding sequence" refers to a DNA sequence that codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to, promoters, translation leader sequences, introns, and polyadenylation recognition sequences.

"Promoter" refers to a DNA sequence capable of controlling the expression of a coding sequence or functional RNA. The promoter sequence consists of proximal and more distal upstream elements, the latter elements often referred to as enhancers. Accordingly, an "enhancer" is a DNA sequence which can stimulate promoter activity and may be an innate element of the promoter or a heterologous element inserted to enhance the level or tissue-specificity of a promoter. Promoter sequences can also be located within the transcribed portions of genes, and/or downstream of the transcribed sequences. Promoters may be derived in their entirety from a native gene, or be composed of different elements derived from different promoters found in nature, or even comprise synthetic DNA segments. It is understood by those skilled in the art that different promoters may direct the expression of an isolated nucleic acid fragment in different tissues or cell types, or at different stages of development, or in response to different environmental conditions. Promoters which cause an isolated nucleic acid fragment to be expressed in most cell types at most times are commonly referred to as "constitutive promoters". New promoters of various types useful in plant cells are constantly being discovered; numerous examples may be found in the compilation by Okamuro and Goldberg, (1989) *Biochemistry of Plants 15:*1-82.

It is further recognized that since in most cases the exact boundaries of regulatory sequences have not been completely defined, DNA fragments of some variation may have identical promoter activity. As used herein, "substantially similar and functionally equivalent subfragment of a promoter" refers to a portion or subsequence of a promoter sequence which is capable of controlling the expression of a coding sequence or functional RNA.

Specific examples of promoters that may be useful in expressing the nucleic acid fragments of the invention include, but are not limited to, the promoters disclosed in this application (SEQ ID NO's: 1,2,3 and 4).

An "intron" is an intervening sequence in a gene that does not encode a portion of the protein sequence. Thus, such sequences are transcribed into RNA but are then excised and are not translated. The term is also used for the excised RNA sequences.

An "exon" is a portion of the sequence of a gene that is transcribed and is found in the mature messenger RNA derived from the gene, but is not necessarily a part of the sequence that encodes the final gene product.

The term "deduced nucleotide sequence" refers to a DNA sequence after removal of intervening sequences, based on homology to other DNA sequences encoding the same protein.

The term "deduced amino acid sequence" refers to a polypeptide sequence derived from a DNA sequence after removal of intervening sequences, based on homology to other proteins encoded by DNA sequences encoding the same protein.

The term "translation leader sequence" refers to a DNA sequence located between the promoter sequence of a gene and the coding sequence. The translation leader sequence is present in the fully processed mRNA upstream of the translation start sequence. The translation leader sequence may affect processing of the primary transcript to mRNA, mRNA stability or translation efficiency. Examples of translation leader sequences have been described (Turner, R. and Foster, G. D. (1995) *Molecular Biotechnology 3*:225).

The "3' non-coding sequences" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al., (1989) *Plant Cell 1*:671-680.

"RNA transcript" refers to the product resulting from RNA polymerase-catalyzed transcription of a DNA sequence. When the RNA transcript is a perfect complementary copy of the DNA sequence, it is referred to as the primary transcript or it may be a RNA sequence derived from post-transcriptional processing of the primary transcript and is referred to as the mature RNA. "Messenger RNA (mRNA)" refers to the RNA that is without introns and that can be translated into protein by the cell. "cDNA" refers to a DNA that is complementary to and synthesized from a mRNA template using the enzyme reverse transcriptase. The cDNA can be single-stranded or converted into the double-stranded form using the Klenow fragment of DNA polymerase I. "Sense" RNA refers to RNA transcript that includes the mRNA and can be translated into protein within a cell or *in vitro.* "Antisense RNA" refers to an RNA transcript that is complementary to all or part of a target primary transcript or mRNA and that blocks the expression of a target isolated nucleic acid fragment (U.S. Patent No. 5,107,065). The complementarity of an antisense RNA may be with any part of the specific gene transcript, i.e., at the 5' non-coding sequence, 3' non-coding sequence, introns, or the coding sequence. "Functional RNA" refers to antisense RNA, ribozyme RNA, or other RNA that may not be translated but yet has an effect on cellular processes. The terms "complement" and "reverse complement" are used interchangeably herein with respect to mRNA transcripts, and are meant to define the antisense RNA of the message.

The term "endogenous RNA" refers to any RNA which is encoded by any nucleic acid sequence present in the genome of the host, whether naturally-occurring or non-naturally occurring, i.e., introduced by recombinant means, mutagenesis, etc.

The term "non-naturally occurring" means artificial, not consistent with what is normally found in nature.

The term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., that the coding sequence is under the transcriptional control of the promoter). Coding sequences can be operably linked to regulatory sequences in a sense or antisense orientation. In another example, the complementary RNA regions of the invention can be operably linked, either directly or indirectly, 5' to the target mRNA, or 3' to the target mRNA, or within the target mRNA, or a first complementary region is 5' and its complement is 3' to the target mRNA.

The term "expression", as used herein, refers to the production of a functional end-product. Expression of an isolated nucleic acid fragment involves transcription of the isolated nucleic acid fragment and translation of the mRNA into a precursor or mature protein. "Antisense inhibition" refers to the production of antisense RNA transcripts capable of suppressing the expression of the target protein. "Co-suppression" refers to the production of sense RNA transcripts capable of suppressing the expression of identical or substantially similar foreign or endogenous genes (U.S. Patent No. 5,231,020).

"Mature" protein refers to a post-translationally processed polypeptide; i.e., one from which any pre- or propeptides present in the primary translation product have been removed. "Precursor" protein refers to the primary product of translation of mRNA; i.e., with pre- and propeptides still present. Pre- and propeptides may be but are not limited to intracellular localization signals.

"Stable transformation" refers to the transfer of a nucleic acid fragment into a genome of a host organism, including both nuclear and organellar genomes, resulting in genetically stable inheritance. In contrast, "transient transformation" refers to the transfer of a nucleic acid fragment into the nucleus, or DNA-containing organelle, of a host organism resulting in gene expression without integration or stable inheritance. Host organisms containing the transformed nucleic acid fragments are referred to as "transgenic" organisms. The preferred method of cell transformation of rice, corn and other monocots is the use of particle-accelerated or "gene gun" transformation technology (Klein et al., (1987) Nature (London) 327:70-73; U.S. Patent No. 4,945,050), or an Agrobacterium-mediated method using an appropriate Ti plasmid containing the transgene (Ishida Y. et al., 1996, Nature Biotech. 14:745-750). The term "transformation" as used herein refers to both stable transformation and transient transformation.

Standard recombinant DNA and molecular cloning techniques used herein are well known in the art and are described more fully in Sambrook, J., Fritsch, E.F. and Maniatis, T. Molecular Cloning: A Laboratory Manual; Cold Spring Harbor Laboratory Press: Cold Spring Harbor, 1989 (hereinafter "Sambrook").

The term "recombinant" refers to an artificial combination of two otherwise separated segments of sequence, e.g., by chemical synthesis or by the manipulation of isolated segments of nucleic acids by genetic engineering techniques.

"PCR" or "Polymerase Chain Reaction" is a technique for the synthesis of large quantities of specific DNA segments, consists of a series of repetitive cycles (Perkin Elmer Cetus Instruments, Norwalk, CT). Typically, the double stranded DNA is heat denatured, the two primers complementary to the 3' boundaries of the target segment are annealed at low temperature and then extended at an intermediate temperature. One set of these three consecutive steps is referred to as a cycle.

Polymerase chain reaction ("PCR") is a powerful technique used to amplify DNA millions of fold, by repeated replication of a template, in a short period of time. (Mullis et al, Cold Spring Harbor Symp. Quant. Biol. 51:263-273 (1986); Erlich et al, European Patent Application 50,424; European Patent Application 84,796; European Patent Application 258,017, European Patent Application 237,362; Mullis, European Patent Application 201,184, Mullis et al U.S. Patent No. 4,683,202; Erlich, U.S. Patent No. 4,582,788; and Saiki et al, U.S. Patent No. 4,683,194). The process utilizes sets of specific in vitro synthesized oligonucleotides to prime DNA synthesis. The design of the primers is dependent upon the sequences of DNA that are desired to be analyzed. The technique is carried out through many cycles (usually 20-50) of melting the template at high temperature, allowing the primers to anneal to complementary sequences within the template and then replicating the template with DNA polymerase.

The products of PCR reactions are analyzed by separation in agarose gels followed by ethidium bromide staining and visualization with UV transillumination. Alternatively, radioactive dNTPs can be added to the PCR in order to incorporate label into the products. In this case the products of PCR are visualized by exposure of the gel to x-ray film. The added advantage of radiolabeling PCR products is that the levels of individual amplification products can be quantitated.

The terms "recombinant construct", "expression construct" and "recombinant expression construct" are used interchangeably herein. These terms refer to a functional unit of genetic material that can be inserted into the genome of a cell using standard methodology well known to one skilled in the art. Such construct may be itself or may be used in conjunction with a vector. If a vector is used then the choice of vector is dependent upon the method that will be used to transform host plants as is well known to those skilled in the art. For example, a plasmid vector can be used. The skilled artisan is well aware of the genetic elements that must be present on the vector in order to successfully transform, select and propagate host cells comprising any of the isolated nucleic acid fragments of the invention. The skilled artisan will also recognize that different independent transformation events will result in different levels and patterns of expression (Jones et al., (1985) EMBO J. 4:2411-2418; De Almeida et al., (1989) Mol. Gen. Genetics 218:78-86), and thus that multiple events must be screened in order to obtain lines displaying the desired expression level and pattern. Such screening may be accomplished by Southern analysis of DNA, Northern analysis of mRNA expression, Western analysis of protein expression, or phenotypic analysis.

Co-suppression constructs in plants previously have been designed by focusing on overexpression of a nucleic acid sequence having homology to an endogenous mRNA, in the sense orientation, which results in the reduction of all RNA having homology to the overexpressed sequence (see Vaucheret et al. (1998) Plant J 16:651-659; and Gura (2000) Nature 404:804-808). The overall efficiency of this phenomenon is low, and the extent of the RNA reduction is widely variable. Recent work has described the use of "hairpin" structures that incorporate all, or part, of an mRNA encoding sequence in a complementary orientation that results in a potential "stem-loop" structure for the expressed RNA (PCT Publication WO 99/53050 published on October 21, 1999). This increases the frequency of co-suppression in the recovered transgenic plants. Another variation describes the use of plant viral sequences to direct the suppression, or "silencing", of proximal mRNA encoding sequences (PCT Publication WO 98/36083 published on August 20, 1998). Both of these co-suppressing phenomena have not been elucidated mechanistically, although recent genetic evidence has begun to unravel this complex situation (Elmayan et al. (1998) Plant Cell 10:1747-1757).

In one aspect, this invention includes an isolated polynucleotide comprising a nucleotide sequence encoding a polypeptide required for proper root formation, wherein the absence of or altered levels of the polypeptide in a plant results in the elimination of or altered levels of formation of one or more of the nodal roots,
wherein the polypeptide has an amino acid sequence of at least 70%, 75%, 80%, 85%, 90%, 95%, or 99% sequence identity, based on the Clustal V method of alignment, when compared to one of SEQ ID NO:6, 8 , 30, or 38. The polypeptide may also comprise SEQ ID NO:6, 8 30, or 38, and the nucleotide sequence may comprise SEQ ID NO:5, 7, 29, or 37.

Also included in the present invention is a complement of any of the foregoing nucleotide sequences, wherein the complement and the nucleotide sequence consist of the same number of nucleotides and are 100% complementary.

As used herein, "proper root formation" means a root system that exhibits formation of all nodal roots (lateral seminal, crown, and brace roots). A polypeptide is required for proper root formation in that the absence of or altered levels of the polypeptide in a plant results in the elimination of or altered levels of formation of one or more of the nodal roots.

In another aspect, this invention includes isolated polynucleotides as described herein (or complements), wherein the nucleotide sequence comprises a sequence which encodes at least two, three, four, or five motifs selected from group consisting of SEQ ID NOs:9, 10, 11, 12 and 13, wherein said motif is a substantially conserved subsequence.

"Motifs" or "subsequences" refer to short regions of conserved sequences of nucleic acids or amino acids that comprise part of a longer sequence. For example, it is expected that such conserved subsequences (for example SEQ ID NOs: 9,10,11,12 and 13) would be important for function, and could be used to identify new homologues of RTCS-homologues in plants. It is expected that some or all of the elements may be found in an RTCS-homologue. Also, it is expected that at least one or two of the conserved amino acids in any given motif may differ in a true RTCS-homologue.

In another aspect, a polynucleotide of this invention or a functionally equivalent subfragment thereof is useful in antisense inhibition or cosuppression of expression of nucleic acid sequences encoding proteins required for proper root formation of the invention, most preferably in antisense inhibition or cosuppression of an endogenous RTCS or heterologous RTCS gene.

Protocols for antisense inhibition or co-suppression are well known to those skilled in the art and are described above.

In still a further aspect, this invention includes an isolated nucleic acid fragment comprising (a) a promoter consisting essentially of SEQ ID NO:2, 3 or 4, or (b) a substantially similar and functionally equivalent subfragment of said promoter.

Also of interest are recombinant DNA constructs comprising any of the above-identified isolated nucleic acid fragments or isolated polynucleotides or complements thereof or parts of such fragments or complements, operably linked to at least one regulatory sequence.

Plants, plant tissue or plant cells comprising such recombinant DNA constructs in their genome are also within the scope of this invention. Transformation methods are well known to those skilled in the art and are described above. Any plant, dicot or monocot can be transformed with such recombinant DNA constructs.

Examples of monocots include, but are not limited to, corn, wheat, rice, sorghum, millet, barley, palm, lily, *Alstroemeria,* rye, and oat. Examples of dicots include, but are not limited to, soybean, rape, sunflower, canola, grape, guayule, columbine, cotton, tobacco, peas, beans, flax, safflower, alfalfa.

Plant tissue includes differentiated and undifferentiated tissues or plants, including but not limited to, roots, stems, shoots, leaves, pollen, seeds, tumor tissue, and various forms of cells and culture such as single cells, protoplasm, embryos, and callus tissue. The plant tissue may in plant or in organ, tissue or cell culture.

In another aspect, this invention includes a method of altering root structure during plant development, comprising:
(a) transforming a plant with a recombinant DNA construct of the invention;
(b) growing the transformed plant under conditions suitable for the expression of the recombinant DNA construct; and
(c) selecting those transformed plants having altered root structure.

As used herein, altering root structure includes altering one or more of the nodal roots (lateral seminal, crown, and brace roots). Alterations may include alterations in the level of growth of any or all of the nodal roots or alterations in root architecture. The alterations may result in increased or decreased changes.

The regeneration, development, and cultivation of plants from single plant protoplast transformants or from various transformed explants is well known in the art (Weissbach and Weissbach, In: Methods for Plant Molecular Biology, (Eds.), Academic Press, Inc. San Diego, CA, (1988)). This regeneration and growth process typically includes the steps of selection of transformed cells, culturing those individualized cells through the usual stages of embryonic development through the rooted plantlet stage. Transgenic embryos and seeds are similarly regenerated. The resulting transgenic rooted shoots are thereafter planted in an appropriate plant growth medium such as soil.

The development or regeneration of plants containing the foreign, exogenous isolated nucleic acid fragment that encodes a protein of interest is well known in the art. Preferably, the regenerated plants are self-pollinated to provide homozygous transgenic plants. Otherwise, pollen obtained from the regenerated plants is crossed to seed-grown plants of agronomically important lines. Conversely, pollen from plants of these important lines is used to pollinate regenerated plants. A transgenic plant of the present invention containing a desired polypeptide is cultivated using methods well known to one skilled in the art.

There are a variety of methods for the regeneration of plants from plant tissue.

The particular method of regeneration will depend on the starting plant tissue and the particular plant species to be regenerated.

Methods for transforming dicots, primarily by use of *Agrobacterium tumefaciens,* and obtaining transgenic plants have been published for cotton (U.S. Patent No. 5,004,863, U.S. Patent No. 5,159,135, U.S. Patent No. 5,518, 908); soybean (U.S. Patent No. 5,569,834, U.S. Patent No. 5,416,011, McCabe et. al., BiolTechnology 6:923 (1988), Christou et al., Plant Physiol. 87:671-674 (1988)); *Brassica* (U.S. Patent No. 5,463,174); peanut (Cheng et al., Plant Cell Rep. 15:653-657 (1996), McKently et al., Plant Cell Rep. 14:699-703 (1995)); papaya; and pea (Grant et al., Plant Cell Rep. 15:254-258, (1995)).

Transformation of monocotyledons using electroporation, particle bombardment, and *Agrobacterium* have also been reported. Transformation and plant regeneration have been achieved in asparagus (Bytebier et al., Proc. Natl. Acad. Sci. (USA) 84:5354, (1987)); barley (Wan and Lemaux, Plant Physiol 104:37 (1994)); *Zea mays* (Rhodes et al., Science 240:204 (1988), Gordon-Kamm et al., Plant Cell 2:603-618 (1990), Fromm et al., BiolTechnology 8:833 (1990), Koziel et al., BiolTechnology 11: 194, (1993), Armstrong et al., Crop Science 35:550-557 (1995)); oat (Somers et al., BiolTechnology 10: 15 89 (1992)); orchard grass (Horn et al., Plant Cell Rep. 7:469 (1988)); rice (Toriyama et al., Theor Appl. Genet. 205:34, (1986); Part et al., Plant Mol. Biol. 32:1135-1148, (1996); Abedinia et al., Aust. J. Plant Physiol. 24:133-141 (1997); Zhang and Wu, Theor. Appl. Genet. 76:835 (1988); Zhang et al. Plant Cell Rep. 7:379, (1988); Battraw and Hall, Plant Sci. 86:191-202 (1992); Christou et al., Bio/Technology 9:957 (1991)); rye (De la Pena et al., Nature 325:274 (1987)); sugarcane (Bower and Birch, Plant J. 2:409 (1992)); tall fescue (Wang et al., Bio/Technology 10:691 (1992)), and wheat (Vasil et al., BiolTechnology 10:667 (1992); U.S. Patent No. 5,631,152).

Assays for gene expression based on the transient expression of cloned nucleic acid constructs have been developed by introducing the nucleic acid molecules into plant cells by polyethylene glycol treatment, electroporation, or particle bombardment (Marcotte et al., Nature 335:454-457 (1988); Marcotte et al., Plant Cell 1:523-532 (1989); McCarty et al., Cell 66:895-905 (1991); Hattori et al., Genes Dev. 6:609-618 (1992); Goff et al., EMBO J. 9:2517-2522 (1990)).

Transient expression systems may be used to functionally dissect isolated nucleic acid fragment constructs (see generally, Maliga et al., Methods in Plant Molecular Biology, Cold Spring Harbor Press (1995)). It is understood that any of the nucleic acid molecules of the present invention can be introduced into a plant cell in a permanent or transient manner in combination with other genetic elements such as vectors, promoters, enhancers etc.

In addition to the above discussed procedures, practitioners are familiar with the standard resource materials which describe specific conditions and procedures for the construction, manipulation and isolation of macromolecules (e.g., DNA molecules, plasmids, etc.), generation of recombinant organisms and the screening and isolating of clones, (see for example, Sambrook et al., Molecular Cloning: A Laboratory Manual, Cold Spring Harbor Press (1989); Maliga et al., Methods in Plant Molecular Biology, Cold Spring Harbor Press (1995); Birren et al., Genome Analysis: Detecting Genes, 1, Cold Spring Harbor, New York (1998); Birren et al., Genome Analysis: Analyzing DNA, 2, Cold Spring Harbor, New York (1998); Plant Molecular Biology: A Laboratory Manual, eds. Clark, Springer, New York (1997)).

In a still further aspect, this invention includes a method to isolate nucleic acid fragments encoding polypeptides associated with altering root structure during plant development, which comprises:
(a) comparing SEQ ID NOs: 6, 8, 30, or 38 with other-polypeptide sequences associated with altering root structure during plant development;
(b) identifying the conserved sequences(s) of 4 or more amino acids obtained in step (a);
(c) making region-specific nucleotide probe(s) or oligomer(s) based on the conserved sequences identified in step (b); and
(d) using the nucleotide probe(s) or oligomer(s) of step (c) to isolate sequences associated with altering root structure during plant development by sequence dependent protocols.

Examples of conserved sequence elements that would be useful in identifying other plant sequences associated with altering root structure during plant development can be found in the group comprising, but not limited to, the nucleotides encoding the polypeptides of SEQ ID NO:9, 10,11, 12, and 13.

Also disclosed is a method of mapping genetic variations related to altering root structure during plant development comprising:
(a) crossing two plant varieties; and
(b) evaluating genetic variations with respect to:
   (i) a nucleic acid sequence selected from the group consisting of SEQ ID NO:5,7, 29, or 37; or
   (ii) a nucleic acid sequence encoding a polypeptide selected from the group consisting of SEQ ID NO: 6, 8, 30, or 38 in progeny plants resulting from the cross of step (a) wherein the evaluation is made using a method selected from the group consisting of: RFLP analysis, SNP analysis, and PCR-based analysis.

Also disclosed is a method of molecular breeding to obtain altered root formation:
(a) crossing two plant varieties; and
(b) evaluating genetic variations with respect to:
   (i) a nucleic acid sequence selected from the group consisting of SEQ ID NO: 5,7 ,29, or 37; or
   (ii) a nucleic acid sequence encoding a polypeptide selected from the group consisting of SEQ ID NO: 6,8, 30, or 38
      in progeny plants resulting from the cross of step (a) wherein the evaluation is made using a method selected from the group consisting of: RFLP analysis, SNP analysis, and PCR-based analysis.

The terms "mapping genetic variation" or "mapping genetic variability" are used interchangeably and define the process of identifying changes in DNA sequence, whether from natural or induced causes, within a genetic region that differentiates between different plant lines, cultivars, varieties, families, or species. The genetic variability at a particular locus (gene) due to even minor base changes can alter the pattern of restriction enzyme digestion fragments that can be generated. Pathogenic alterations to the genotype can be due to deletions or insertions within the gene being analyzed or even single nucleotide substitutions that can create or delete a restriction enzyme recognition site. RFLP analysis takes advantage of this and utilizes Southern blotting with a probe corresponding to the isolated nucleic acid fragment of interest.

Thus, if a polymorphism (i.e., a commonly occurring variation in a gene or segment of DNA; also, the existence of several forms of a gene (alleles) in the same species) creates or destroys a restriction endonuclease cleavage site, or if it results in the loss or insertion of DNA (e.g., a variable nucleotide tandem repeat (VNTR) polymorphism), it will alter the size or profile of the DNA fragments that are generated by digestion with that restriction endonuclease. As such, individuals that possess a variant sequence can be distinguished from those having the original sequence by restriction fragment analysis. Polymorphisms that can be identified in this manner are termed "restriction fragment length polymorphisms: ("RFLPs"). RFLPs have been widely used in human and plant genetic analyses (Glassberg, UK Patent Application 2135774; Skolnick et al, Cytogen. Cell Genet. 32:58-67 (1982); Botstein et al, Ann. J. Hum. Genet. 32:314-331 (1980); Fischer et al (PCT Application WO 90/13668; Uhlen, PCT Application WO 90/11369).

A central attribute of "single nucleotide polymorphisms" or "SNPs" is that the site of the polymorphism is at a single nucleotide. SNPs have certain reported advantages over RFLPs or VNTRs. First, SNPs are more stable than other classes of polymorphisms. Their spontaneous mutation rate is approximately 10⁻⁹ (Kornberg, DNA Replication, W.H. Freeman & Co., San Francisco, 1980), approximately, 1,000 times less frequent than VNTRs (U.S. Patent 5,679,524). Second, SNPs occur at greater frequency, and with greater uniformity than RFLPs and VNTRs. As SNPs result from sequence variation, new polymorphisms can be identified by sequencing random genomic or cDNA molecules. SNPs can also result from deletions, point mutations and insertions. Any single base alteration, whatever the cause, can be a SNP. The greater frequency of SNPs means that they can be more readily identified than the other classes of polymorphisms.

SNPs can be characterized using any of a variety of methods. Such methods include the direct or indirect sequencing of the site, the use of restriction enzymes where the respective alleles of the site create or destroy a restriction site, the use of allele-specific hybridization probes, the use of antibodies that are specific for the proteins encoded by the different alleles of the polymorphism or by other biochemical interpretation. SNPs can be sequenced by a number of methods. Two basic methods may be used for DNA sequencing, the chain termination method of Sanger et al, Proc. Natl. Acad. Sci. (U.S.A.) 74:5463-5467 (1977), and the chemical degradation method of Maxam and Gilbert, Proc. Natl. Acad. Sci. (U.S.A.) 74: 560-564 (1977).

Furthermore, single point mutations can be detected by modified PCR techniques such as the ligase chain reaction ("LCR") and PCR-single strand conformational polymorphisms ("PCR-SSCP") analysis. The PCR technique can also be used to identify the level of expression of genes in extremely small samples of material, e.g., tissues or cells from a body. The technique is termed reverse transcription-PCR ("RT-PCR").

The term "molecular breeding" defines the process of tracking molecular markers during the breeding process. It is common for the molecular markers to be linked to phenotypic traits that are desirable. By following the segregation of the molecular marker or genetic trait, instead of scoring for a phenotype, the breeding process can be accelerated by growing fewer plants and eliminating assaying or visual inspection for phenotypic variation. The molecular markers useful in this process include, but are not limited to, any marker useful in identifying mapable genetic variations previously mentioned, as well as any closely linked genes that display synteny across plant species. The term "synteny" refers to the conservation of gene placement/order on chromosomes between different organisms. This means that two or more genetic loci, that may or may not be closely linked, are found on the same chromosome among different species. Another term for synteny is "genome colinearity".

### EXAMPLES

The present invention is further defined in the following Examples, in which parts and percentages are by weight and degrees are Celsius, unless otherwise stated. It should be understood that these Examples, while indicating preferred embodiments of the invention, are given by way of illustration only.

### EXAMPLE 1

### Map-based Cloning of RTCS

In order to map the rtcs mutation, two mapping populations and their corresponding corn seeds, segregating for the rtcs gene, were utilized. The first mapping populations consisted of 1591 plants derived from a F1 cross between inbred line DK105, carrying the rtcs mutation, and the inbred line B73. The second mapping populations consisted of 475 plants derived from a F1 cross between inbred line DK105, carrying the rtcs mutation, and the inbred line Mo17.

Homozygous *rtcs*/*rtcs* plants were scored as completely lodged plants when grown in the field for 40 days or more. In the B73 and Mo17 F2 polpulations, 388 and 61 mutant plants were retrieved, respectively. These plants were selected for fine mapping of the rtcs locus.

DNA was extracted from those plants using standard molecular biology procedures.

To obtain F2 plants that carry recombination near the *rtcs* locus, public PCR-based DNA markers (SSRs) present in the Maize Genetics and Genomic Database (MaizeGDB), were used. When these were not available, CAP (allele-specific PCR primers) markers were developed from the DuPont proprietary sequences of BAC (Bacterial Artificial Chromosome) clones of known map positions. Both CAP and SSR primers were used in a PCR reaction containing 25ng of DNA.

Flanking SSR marker BNLG 1014 [BNLG 1014 forward primer (SEQ ID NO:14), BNLG 1014 reverse primer (SEQ ID NO: 15)] and BNLG 1429 [BNLG 1429 forward primer (SEQ ID NO:16), BNLG 1429 reverse primer (SEQ ID NO: 17)] were retrieved from the MaizeGDB. These markers are localized at 82.80 cM and 143.50 cM of Chromosome 1 respectively, based on the public map IBM2 neighbors 1.

SSR markers were analyzed using a Perkin Elmer ABI 3700 machine. PCR was performed in 20 ul, using Qiagen Hot start mix (Qiagen), following the manufacturer instructions, and using one of the two amplifying primers labeled with a specific fluorochrome.

When using these 2 primers on 367 *rtcs* plants, a total of 97 recombinants were obtained, 34 with marker BNLG 1014 and 63 from marker BNLG1429, indicating that *rtcs* was closer to BNLG 1014.

In order to obtain genetic markers closer to *rtcs,* more primers were retrieved from the Maize GDB based on their position along chromosome 1 and tested on the same number of individuals. In particular, markers UMC1685 (UMC1685 forward primer (SEQ ID NO: 18), UMC 1685 reverse primer (SEQ ID NO: 19)] gave 7 recombinants and marker UMC1660 [UMC1660 forward primer (SEQ ID NO:20), UMC 1660 reverse primer (SEQ ID NO:21)] gave 11 recombinants indicating a distance of 0.95 cM and 1.5 cM from the rtcs locus respectively.

Marker UMC1685 and UMC 1660 have been physically positioned by hybridization onto a single maize contig, named 1871 (Dupont Genomix database). The physical distance between the two markers encompasses approximately 10 BACs.

Based on this information, new CAP markers were designed using available BAC-end sequences of the BACs constituting the region of contig 1871 surrounded by markers UMC 1685 and 1660.

Cap marker b104.i24 [b104.i24 forward primer (SEQ ID NO:22), b104.i24 reverse primer (SEQ ID NO:23)] was designed based on the BAC-end sequence of clone BAC 104h.i24. This primer set amplifies a region of 450 bp, showing polymorphism between B73 and DK105 after restriction with the 4-cutter enzyme Hhal.

CAP marker amplifications were performed in a 25 ul PCR reaction using the Qiagen HotStart mix and 25 ng DNA. The thermal cycle conditions were: 95°C 15min (1 cycle), 94°C 45 sec, 56°C 45 sec, 72°C 45 sec, (35 cycles) 72°C 7 min.

3 ul of the amplification product was used for a restriction digest (total volume of 15 ul) with the 4-cutter restriction enzyme Hhal (Promega). Restriction reaction was carried out at 37C for one hour. Restricted amplification products were examined on 2.5 % agarose gels. By screening the 18 previously obtained recombinants with this primers set, only 7 recombination breakpoint were found, on the same side of the marker UMC1160, meaning that rtcs lies exactly in the middle between markers UMC 1685 and b104.124.

Cap marker b74.m9 [ b74.m9 forward primer (SEQ ID NO:24), b74.m9 reverse primer (SEQ ID NO:25)] was designed based on the BAC-end sequence of clone BAC b74a.m9. This primer set amplifies a region of 313 bp, showing polymorphism between B73 and DK105 after restriction with the 4-cutter enzyme Haelll. This CAP marker allowed us to narrow down the genomic region containing the rtcs locus between marker UMC 1685 (at a distance of 0.95 cM, with 7 recombinant plants), located on BAC clone 35.m15 and marker b74.m9, corresponding to the end of the BAC clone b74.m9 at a distance of 0.13 cM (one recombination breakpoint). The 2 BAC clones are overlapping.

### EXAMPLE 2

### Identification of the RTCS Gene

In order to identify the *RTCS* gene that was mapped to the region comprising the two overlapping BAC clones, BAC 74.m9 was sequenced. The 6kb fragment of Bac74.m9 containing the RTCS gene is shown in SEQ ID NO:1. For the purpose, BAC DNA was nebulized using high-pressure nitrogen gas as described in Roe et al. 1996 (Roe et al. (1996) "DNA isolation and Sequencing" John Wiley and Sons, New York).

The estimated 150 Kb of sequence of BAC 74.m9 was searched for the presence of open reading frames, and 4 regions, showing similarities to genes filed in Genbank as well as in the DuPont proprietary EST database, were identified. These regions correspond to the rice genes annotated as OSJNBb0050N02.6, OSJNBb0050N02.7, OSJNBb0050N02.9 and OSJNBb0050N02.10 of the rice BAC OSJNBb0050N02, annotated in Genebank as AC105734. These rice genes have been annotated as "hypothetical proteins". The corresponding corn ESTs are cds3f.pk004.j15, cen3n.pk0159.d12, cr1n.pk0028.h3a:fis and cpf1c.pk006.d18a:fis (from DuPont proprietary EST database). These genes were selected because they were in the middle of the recombination data.

The candidate genes were then PCR amplified from genomic DNA of the wild type (DK105) and the mutant genotypes *(rtcs),* and the sequences compared. By comparing the sequences of (1) a gene encoding a corn homologue (amplified by PCR from the wild type and the mutant using the sequence from bac 74.m9 (SEQ ID NO:1) corresponding to the rice gene OSJNBb0050N02.10 (which is annotated as "hypothetical protein" in Genebank) and (2) one of the arabidopsis "LOB domain" gene family members (Gl No. 2761471) a mutation was found in the mutant allele. The *rtcs* allele carries a 5bp insertion at position 227bp from the starting ATG, which causes a frameshift and introduces a premature stop codon.

### EXAMPLE 3

### Cloning the RTCS cDNA

Total RNA can be extracted from developing maize using a TRIazol^{®} Reagent obtained from Life Technologies Inc., Rockville, MD, 20849 (GIBCO-BRL) that contains phenol and guanidine thiocyanate. Poly A mRNA can be purified from total RNA with mRNA Purification kits obtained from Amersham Pharmacia Biotech Inc., Piscataway, NJ, 08855, which consists of oligo (dT)-cellulose spin columns. To make the cDNA library, 5.5 ug of polyA RNA can be used for cDNA synthesis kits, which can be obtained from Stratagene, La Jolla, CA, 92037. Superscript^{®} reverse transcriptase can be obtained from Life Technologies Inc., Rockville, MD, 20849 (GIBCO-BRL). BRL cDNA Size Fraction Columns (GIBCO-BRL) can be used to fractionate the cDNA by size, fractions can be precipitated, resuspended and ligated with 1 ug of the Uni-ZAP XR vector. After ligation it can be packaged in Gigapack III Gold^{®} packaging extract obtained from Stratagene, La Jolla, CA, 92037. The unamplified library titer can be estimated. An appropriate amount can be used for amplification purposes to produce amplified cDNA.
Screening for the *RTCS* cDNA follows standard protocols well known to those skilled in the art (Ausubel et al. 1993, "Current Protocols in Molecular Biology" John Wiley & Sons, USA, or Sambrook et al. 1989. Molecular Cloning: A Laboratory Manual. Cold Spring Harbor Laboratory Press). Briefly, 1.5 X 10⁶ phage clones can be plated, then transferred to nylon membranes, which then will be subjected to hybridization with radioactively labeled *RTCS* probe. Positives are isolated and examined for their identity as RTCS cDNAs through PCR with RTCS-specific primers. The longest cDNA clones that give positive results from the PCR reaction are isolated and sequenced.

### EXAMPLE 4

### Cloning and isolation of a full length RTCS cDNA clone from corn

A lambda cDNA library (cdr1f) was prepared from 15 days old B73 nodal roots. The library was screened using a radioactive probe generated by PCR amplification of the bac clone BAC74.m9 using the the primers shown in SEQ ID NO.: 31 and 32 and as described in Example 3.The full length RTCS cDNA clone was retrieved after screening 1.8x10⁶ lambda clones. The sequence of the full length corn RTCS cDNA clone is identical to SEQ ID NO:29, except for one nucleotide difference which does not change the encoded amino acid sequence, which is identical to SEQ ID NO:30.

### EXAMPLE 5

### Genetic Confirmation of the RTCS gene

The genetic confirmation that the RTCS isolated nucleic acid fragment encodes the polypeptide responsible for altering root structure can be accomplished by transforming *rtcs* mutants with the isolated RTCS cloned sequence.

RTCS homologs from other crop species can also be tested in this system by obtaining full-gene sequences, ligation to an appropriate promoter, such as the RTCS promoter and complementing the maize RTCS mutant.

In order to confirm possible tissue-specific expression of the *RTCS* gene, the presence of the *RTCS* transcript in various tissues can be analyzed by RNA blot analysis and in situ hybridization.

One method for transforming DNA into cells of higher plants that is available to those skilled in the art is high-velocity ballistic bombardment using metal particles coated with the nucleic acid constructs of interest (see Klein et al. Nature (1987) (London) 327:70-73, and see U.S. Patent No. 4,945,050). A Biolistic PDS-1000/He (BioRAD Laboratories, Hercules, CA) can be used for these complementation experiments (see Example 4 for further details). The particle bombardment technique can be used to transform the rtcs mutant with the cloned RTCS wild type sequence [SEQ ID NO:5 or 7], encoding a functional RTCS protein.

The bacterial hygromycin B phosphotransferase (Hpt II) gene from *Streptomyces hygroscopicus* that confers resistance to the antibiotic hygromycin can be used as the selectable marker for the maize transformation. In the vector, pML18, the Hpt II gene can be engineered with the 35S promoter from Cauliflower Mosaic Virus and the termination and polyadenylation signals from the octopine synthase gene of *Agrobacterium tumefaciens.* pML18 was described in WO 97/47731, which was published on December 18, 1997, the disclosure of which is hereby incorporated by reference.

Embryogenic maize callus cultures derived serve as source material for transformation experiments. This material can be generated by germinating sterile maize seeds on a callus initiation media (MS salts, Nitsch and Nitsch vitamins, 1.0 mg/l 2,4-D and 10 µM AgNO₃) in the dark at 27-28°C. Embryogenic callus proliferating from the scutellum of the embryos is then transferred to CM media (N6 salts, Nitsch and Nitsch vitamins, 1 mg/l 2,4-D, Chu et al., 1985, Sci. Sinica 18: 659-668). Callus cultures are maintained on CM by routine sub-culture at two week intervals and used for transformation within 10 weeks of initiation.

Callus can be prepared for transformation by subculturing 0.5-1.0 mm pieces approximately 1 mm apart, arranged in a circular area of about 4 cm in diameter, in the center of a circle of Whatman #541 paper placed on CM media. The plates with callus are incubated in the dark at 27-28°C for 3-5 days. Prior to bombardment, the filters with callus are transferred to CM supplemented with 0.25 M mannitol and 0.25 M sorbitol for 3 hr in the dark. The petri dish lids are then left ajar for 20-45 minutes in a sterile hood to allow moisture on tissue to dissipate.

Each genomic DNA fragment is co-precipitated with pML18 containing the selectable marker for maize transformation onto the surface of gold particles. To accomplish this, a total of 10 µg of DNA at a 2:1 ratio of trait:selectable marker DNAs are added to 50 µl aliquot of gold particles that are resuspended at a concentration of 60 mg ml⁻¹. Calcium chloride (50 µl of a 2.5 M solution) and spermidine (20 µl of a 0.1 M solution) are then added to the gold-DNA suspension as the tube was vortexed for 3 min. The gold particles are centrifuged in a microfuge for 1 sec and the supernatant removed. The gold particles are then washed twice with 1 ml of absolute ethanol and then resuspended in 50 ml of absolute ethanol and sonicated (bath sonicator) for one second to disperse the gold particles. The gold suspension is incubated at -70°C for five minutes and sonicated (bath sonicator) if needed to disperse the particles. Six µl of the DNA-coated gold particles are then loaded onto mylar macrocarrier disks and the ethanol is allowed to evaporate.

At the end of the drying period, a petri dish containing the tissue is placed in the chamber of the PDS-1000/He. The air in the chamber is then evacuated to a vacuum of 28-29 inches Hg. The macrocarrier is accelerated with a helium shock wave using a rupture membrane that bursts when the He pressure in the shock tube reaches 1080-1100 psi. The tissue is placed approximately 8 cm from the stopping screen and the callus was bombarded two times. Two to four plates of tissue are bombarded in this way with the DNA-coated gold particles. Following bombardment, the callus tissue is transferred to CM media without supplemental sorbitol or mannitol.

Within 3-5 days after bombardment the callus tissue is transferred to SM media (CM medium containing 50 mg/l hygromycin). To accomplish this, callus tissue is transferred from plates to sterile 50 ml conical tubes and weighed. Molten top-agar at 40° C is added using 2.5 ml of top agar/100 mg of callus. Callus clumps are broken into fragments of less than 2 mm diameter by repeated dispensing through a 10 ml pipet. Three ml aliquots of the callus suspension are plated onto fresh SM media and the plates are incubated in the dark for 4 weeks at 27-28°C. After 4 weeks, transgenic callus events are identified, transferred to fresh SM plates and grown for an additional 2 weeks in the dark at 27-28°C.

Growing callus can then be transferred to RM1 media (MS salts, Nitsch and Nitsch vitamins, 2% sucrose, 3% sorbitol, 0.4% gelrite +50 ppm hyg B) for 2 weeks in the dark at 25°C. After 2 weeks the callus can be transferred to RM2 media (MS salts, Nitsch and Nitsch vitamins, 3% sucrose, 0.4% gelrite + 50 ppm hyg B) and placed under cool white light (∼40 µEm⁻²s⁻¹) with a 12 hr photoperiod at 25°C and 30-40% humidity. After 2-4 weeks in the light, callus can begin to organize, and form shoots. Shoots can be removed from surrounding callus/media and gently transferred to RM3 media (1/2 x MS salts, Nitsch and Nitsch vitamins, 1% sucrose + 50 ppm hygromycin B) in phytatrays (Sigma Chemical Co., St. Louis, MO) and incubation can be continued using the same conditions as described in the previous step.

Plants can then be transferred from RM3 to 4" pots containing Metro mix 350 after 2-3 weeks, when sufficient root and shoot growth has occurred. The seed obtained from the transgenic plants can be examined for genetic complementation of the rtcs mutation with the wild-type genomic DNA containing the RTCS gene.

### EXAMPLE 6

### Complementation with the corn RTCS and rice RTCS genes

Two constructs are made and used for transformation into corn callus derived from a cross between the inbred line GS3 and the mutant rtcs line, in order to confirm the function of the corn RTCS and the rice RTCS gene.

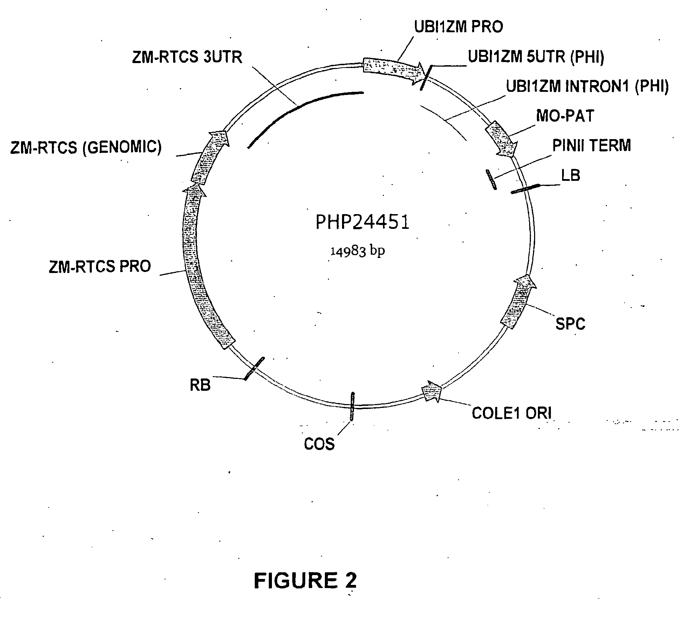

Construct 1: BAC 74.m9 is digested with the restriction enzymes Xmal and HindIII. After gel separation on a 0.7% low melting agarose gel, a fragment of 5408 bp containing the corn RTCS gene, including 2410 bp of endogeneous promoter and 2166 bp of sequence at the 3' end of the RTCS ORF, is recovered and used for ligation into vector PHP20067 digested with Xmal and HindIII, resulting in vector PHP24451 (Fig. 2). The 5408 bp sequence extends from nucleotides 369 to 5776 of SEQ ID NO: 1.

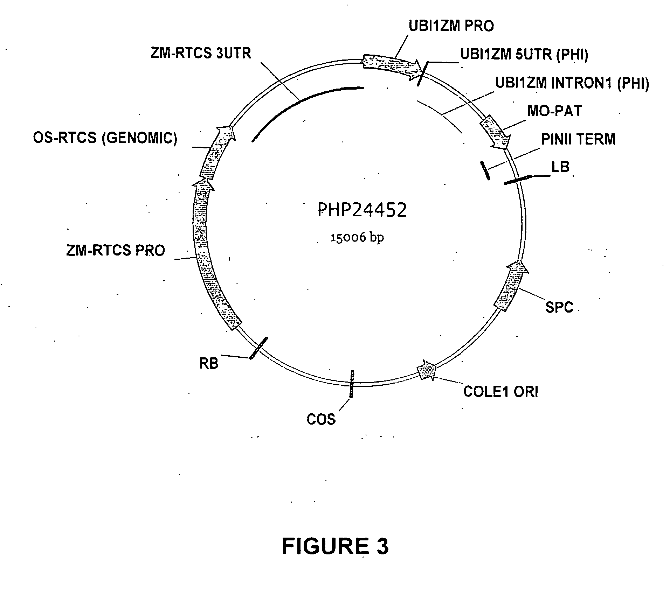

Construct 2: The rice RTCS ORF (SEQ ID NO: 7 ) is used to substitute the corn ORF in the 5408 bp fragment, but the corn promoter region and 3' sequence are maintained in the construct. The construct is prepared as follows. Rice genomic DNA is amplified using the primers shown in SEQ ID NO.: 33 and 34. The PCR fragment is cut with the restriction enzymes Xhol and Drall and purified. The corn RTCS 5408bpXmal- HindIII fragment is cut with Xhol and Drall, and the 3 pieces (corn Xmal- Xhol, rice Xhol-Drall, corn Drall-Hindlll) are ligated in a equimolar reaction. The resulting chimera is cloned into vector PHP20067 digested with Xmal and HindIII, resulting in vector PHP 24452 (Fig. 3).

Vector PHP20067 is constructed using standard molecular biology techniques used by those of skill in the art (Sambrook et al., supra). The plasmid pSB11 can be obtained from Japan Tobacco Inc. (Tokyo, Japan). The construction of pSB11 from pSB21 and the construction of pSB21 from starting vectors is described by Komari et al. (1996, Plant J. 10:165-174). The.plasmid pSB1 (Japan Tobacco Inc., Tokyo, Japan) can further be modified by the addition of a multiple cloning site and a selectable marker gene directing herbicide resistance in plants and plant tissues. In the case of PHP20067, this selectable marker gene comprises a promoter/Intron from the UBIQUITIN gene of Z. mays, a coding sequence for MO-PAT (a maize optimized version of the phosphinothricin acetyltransferase gene from *Streptomyces viridochromogenes*) and a polyadenylation/terminator sequence (PINII TERM) from potato. This gene cassette is ligated into the pSB11-derived vector between the newly introduced multiple cloning site and the LB (Left Border) to generate PHP20067.

After selfing, the regenerated plants will segregate for the presence of the transgene. Only rtcs mutant plants expressing the corn RTCS or rice RTCS gene will show a root phenotype identical to wild type.

### EXAMPLE 7

### Identification of Genomic and cDNA Clones

A maize inbred B73 genomic DNA assembly for the putative RTCS gene was created using BLAST search (Basic Local Alignment Search Tool; Altschul et al. (1993) J. Mol. Biol. 215:403-410) with the maize genomic RTCS sequence from Mo17, contained in SEQ ID NO:1, conducted against the GSS (Genomic Survey Sequences) datasets available in GenBank.

Selected matches (approximately 19 GSS fragments) were downloaded as HTML/text files for import into a sequence assembly program (Sequencher v4.1.4b). Once trimmed of HTML code, these individual fragments were assembled using standard parameters in Sequencher. The contig alignment was then examined to make edits to the consensus sequence. Several of the 19 imported fragments did not assemble and were rejected.

The 13 remaining fragments resulted in a RTCS contig of 3286bp which is shown in SEQ ID NO:28. The accession numbers of the 13 fragments are : gi:32081270, gi:31973186, gi:34279177, gi:34277557, gi:34279170, gi:34277545, gi:32081279, gi:31973192, gi:33915405, gi:33915408, gi:34051270, gi:34051273, gi:34051273.

Clones for cDNAs encoding RTCS-like proteins are identified by conducting BLAST searches. (Basic Local Alignment Search Tool; Altschul et al. (1993) *J. Mol. Biol. 215*:403-410) searches for similarity to sequences contained in the BLAST "nr" database (comprising all non-redundant GenBank CDS translations, sequences derived from the 3-dimensional structure Brookhaven Protein Data Bank, the last major release of the SWISS-PROT protein sequence database, EMBL, and DDBJ databases). The sequence obtained in Example 1 were analyzed for similarity to all publicly available DNA sequences contained in the "nr" database using the BLASTN algorithm provided by the National Center for Biotechnology-Information (NCBI). The DNA sequences were translated in all reading frames and compared for similarity to all publicly available protein sequences contained in the "nr" database using the BLASTX algorithm (Gish and States (1993) *Nat. Genet. 3*:266-272) provided by the NCBI. For convenience, the P-value (probability) of observing a match of a cDNA sequence to a sequence contained in the searched databases merely by chance as calculated by BLAST are reported herein as "pLog" values, which represent the negative of the logarithm of the reported P-value. Accordingly, the greater the pLog value, the greater the likelihood that the sequence and the BLAST "hit" represent homologous proteins.

ESTs submitted for analysis are compared to the genbank database as described above. ESTs that contain sequences more 5- or 3-prime can be found by using the BLASTn algorithm (Altschul et al (1997) *Nucleic Acids Res. 25*:3389-3402.) against the Du Pont proprietary database comparing nucleotide sequences that share common or overlapping regions of sequence homology. Where common or overlapping sequences exist between two or more nucleic acid fragments, the sequences can be assembled into a single contiguous nucleotide sequence, thus extending the original fragment in either the 5 or 3 prime direction. Once the most 5-prime EST is identified, its complete sequence can be determined by Full Insert Sequencing. Homologous genes belonging to different species can be found by comparing the amino acid sequence of a known gene (from either a proprietary source or a public database) against an EST database using the tBLASTn algorithm. The tBLASTn algorithm searches an amino acid query against a nucleotide database that is translated in all 6 reading frames. This search allows for differences in nucleotide codon usage between different species, and for codon degeneracy.

cDNA libraries may be prepared by any one of many methods available. For example, the cDNAs may be introduced into plasmid vectors by first preparing the cDNA libraries in Uni-ZAP™ XR vectors according to the manufacturer's protocol (Stratagene Cloning Systems, La Jolla, CA). The Uni-ZAP™ XR libraries are converted into plasmid libraries according to the protocol provided by Stratagene. Upon conversion, cDNA inserts will be contained in the plasmid vector pBluescript. In addition, the cDNAs may be introduced directly into precut Bluescript II SK(+) vectors (Stratagene) using T4 DNA ligase (New England Biolabs), followed by transfection into DH10B cells according to the manufacturer's protocol (GIBCO BRL Products). Once the cDNA inserts are in plasmid vectors, plasmid DNAs are prepared from randomly picked bacterial colonies containing recombinant pBluescript plasmids, or the insert cDNA sequences are amplified via polymerase chain reaction using primers specific for vector sequences flanking the inserted cDNA sequences. Amplified insert DNAs or plasmid DNAs are sequenced in dye-primer sequencing reactions to generate partial cDNA sequences (expressed sequence tags or "ESTs"; see Adams et al., (1991) *Science 252*:1651-1656). The resulting ESTs are analyzed using a Perkin Elmer Model 377 fluorescent sequencer.Full-insert sequence (FIS) data is generated utilizing a modified transposition protocol. Clones identified for FIS are recovered from archived glycerol stocks as single colonies, and plasmid DNAs are isolated via alkaline lysis. Isolated DNA templates are reacted with vector primed M13 forward and reverse oligonucleotides in a PCR-based sequencing reaction and loaded onto automated sequencers. Confirmation of clone identification is performed by sequence alignment to the original EST sequence from which the FIS request is made.

Confirmed templates are transposed via the Primer Island transposition kit (PE Applied Biosystems, Foster City, CA), which is based upon the *Saccharomyces cerevisiae* Ty1 transposable element (Devine and Boeke (1994) *Nucleic Acids Res. 22*:3765-3772). The *in vitro* transposition system places unique binding sites randomly throughout a population of large DNA molecules. The transposed DNA is then used to transform DH10B electro-competent cells (Gibco BRL/Life Technologies, Rockville, MD) via electroporation. The transposable element contains an additional selectable marker (named DHFR; Fling and Richards (1983) Nucleic Acids Res. 11:5147-5158), allowing for dual selection on agar plates of only those subclones containing the integrated transposon. Multiple subclones are randomly selected from each transposition reaction, plasmid. DNAs are prepared via alkaline lysis, and templates are sequenced (ABI Prism dye-terminator ReadyReaction mix) outward from the transposition event site, utilizing unique primers specific to the binding sites within the transposon.

Sequence data is collected (ABI Prism Collections) and assembled using Phred/Phrap (P. Green, University of Washington, Seattle). Phred/Phrap is a public domain software program which re-reads the ABI sequence data, re-calls the bases, assigns quality values, and writes the base calls and quality values into editable output files. The Phrap sequence assembly program uses these quality values to increase the accuracy of the assembled sequence contigs. Assemblies are viewed by the Consed sequence editor (D. Gordon, University of Washington, Seattle).

### EXAMPLE 8

### Characterization of cDNA Clones Encoding members of RTCS proteins

The BLASTX search using the sequences from clones listed in Table 1 below revealed similarity of the polypeptides encoded by the ORF to the hypothetical protein from rice (NCBI General Identifier No. 27261472, SEQ ID NO: 26) and a putative LOB domain protein from *Arabidopsis* [*Arabidopsis thaliana*] (NCBI General Identifier No. 15230971, which is identical to the amino acid sequence set forth in NCBI General Identifier No. 22331847 (SEQ ID NO:27)).

Table 3 shows the BLAST results for individual ESTs ("EST"), the sequences of the entire cDNA inserts comprising the indicated cDNA clones ("FIS"), the sequences of contigs assembled from two or more ESTs ("Contig"), sequences of contigs assembled from an FIS and one or more ESTs ("Contig*"), or sequences encoding an entire protein derived from an FIS, a contig, or an FIS and PCR ("CGS"):

**TABLE 1**

| BLAST Results for Sequences Encoding the coding region of maize RTCS-like | | | |
|---|---|---|---|
| open reading frame and Polypeptides Homologous To RTCS | | | |
| | | BLAST pLog Score | |
| Sequence | Status | 27261472 | 15230971 |
| PCR product of RTCS gene from bac clone BAC74.m9 | cgs | 243 | 176 |

The data in Table 2 below represents a calculation of the percent identity of the amino acid sequences set forth in SEQ ID NOs:6, 8, 30, and 38 and the hypothetical protein from rice (NCBI General Identifier No. 27261472, SEQ ID NO:26) and the putative LOB domain protein from *Arabidopsis* [*Arabidopsis thaliana*] (NCBI General Identifier No. 22331847, SEQ ID NO:27).

**TABLE 2**

| Percent Identity of Amino Acid Sequences encoded by Nucleotide Sequences of | | |
|---|---|---|
| DNA sequences Encoding the RTCS protein and Polypeptides Homologous To | | |
| RTCS | | |
| | Percent Identity to | |
| SEQ ID NO. | 27261472 | 22331847 |
| 6 | 67.6 | 46.8 |
| 8 | 98.8 | 46.3 |
| 30 | 66.9 | 47.7 |
| 38 | 58.8 | 43.1 |

Sequence alignments and percent identity calculations were performed using the Megalign program of the LASERGENE bioinformatics computing suite (DNASTAR Inc., Madison, WI). Multiple alignment of the sequences was performed using the Clustal method of alignment (Higgins and Sharp (1989) *CABIOS. 5*:151-153) with the default parameters (GAP PENALTY=10, GAP LENGTH PENALTY=10). Default parameters for pairwise alignments using the Clustal method were KTUPLE 1, GAP PENALTY=3, WINDOW=5 and DIAGONALS SAVED=5. Sequence alignments and BLAST scores and probabilities indicate that the nucleic acid fragments comprising the instant sequences encode a substantial portion of a LOB domain protein and share homology with the maize polypeptide encoded by the maize rtcs gene, which gives rise to an altered root structure when mutated.

### EXAMPLE 9

### Isolation of RTCS-LIKE Sequences

Leaves and roots were harvested off 10-day old corn B73 seedlings grown in liquid media consisting of half strength Hoagland salts, plus 7% sucrose added 24 hr prior to sampling. Total RNA was prepared from these samples after they were grounded in liquid nitrogen, using the Tri-PURE kit (Roche, Indianapolis, IN). Using the total RNA as templates, cDNA from the leaf and root samples was prepared using oligo(dT) primers with the Reverse Transcription System (Promega, Madison WI), following manufacturer's instruction.

For isolation of RTCS-LIKE sequences, two gene-specific primers were designed based on the coding sequences of the maize RTCS gene. PCR using the forward primer described in SEQ ID NO: 35, and the reverse primer described in SEQ ID NO:36, was performed in a PTC-200 DNA Engine thermal cycler from MJ Research Inc. (Waltham, Massachusetts) using reagents supplied with the ProofStart DNA Polymerise kit (Qiagen, Valencia, CA). The cDNA isolated from roots and leaves as described above served as a template for the reaction. The following cycle parameters were used: 5 min enzyme activation at 95°C, followed by 30 cycles of 94°C for 15 seconds, 55°C for 30 sec then 72°C for 1 minute. The samples were then held at 72°C for 10 minutes and then at 8°C until further analysis. Each PCR reaction mix was run on 1.0% agarose gel. Only root cDNA produced a PCR product. The PCR band was excised from gel, purified with the Qiaquick Gel Extraction Kit (Qiagen, Valencia, CA). The PCR product was then cloned into the TA cloning vector pCR2.1 (Invitrogen, Carlsbad, CA). The clones were sequenced for verification. The resulting cDNA clone encoding a RTCS-like gene is described in SEQ ID NO: 37. The protein encoded by SEQ ID NO: 37 is shown in SEQ ID NO: 38, and the genomic sequence containing the RTCS-like gene is shown in SEQ ID NO:39.

### EXAMPLE 10

### Identification of a new mutant rtcs allele

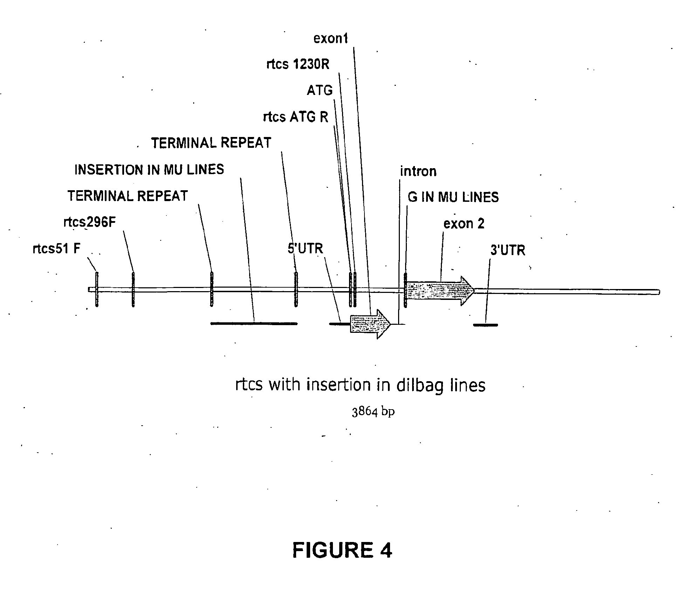

A new mutant rtcs allele has been identified by screening a mu active population. DNA from rtcs plants was extracted and primers 296F (SEQ ID NO.: 40) and 1230R (SEQ ID NO.: 41) were used for PCR amplification. An unexpected PCR product size of 1536bp was obtained, indicating the presence of an insertion in the portion of the gene that was amplified. The mutant plants carry an insertion of 578bp after nucleotide 831 of SEQ ID NO.: 28. Within the 578 additional bp a terminal repeat of 7bp (tcctgct) was found. The mutant plants also carry a A to G mutation at the splicing site of the intron at position 1573 of SEQ ID NO.: 28. The 3864 bp sequence containing the 1546 bp fragment with the mutant sequence is shown in SEQ ID NO.: 42 and Fig.4. Since the phenotype co-segregates perfectly with both the insertion and the mutation, RT-PCR experiments will be carried out in order to clarify whether both or only one of the above-described changes in the mutant can be held accountable for the rtcs phenotype.

### EXAMPLE 11

### Expression of recombinant DNA Constructs in Monocot Cells

A recombinant DNA construct comprising a plant cDNA encoding the instant polypeptides in sense orientation with respect to promoter from the ubiquitin, or CaMV 35S, gene that is located 5' to the cDNA fragment can be constructed. The 3' fragment from the 10kD zein gene [Kirihara et al. (1988) Gene 71:359-370] can be placed 3' to the cDNA fragment. Such constructs are used to overexpress or cosuppress the gene(s) homologous to *RTCS.* It is realized that one skilled in the art could employ different promoters and/or 3'-end sequences to achieve comparable expression results. The construct with the CaMV 35S promoter is made as follows: the transcription termination element is released from the clone by digestion with appropriate restriction enzymes. The fragment is then ligated to appropriate restriction sites of pML141 [PCT Application No. WO 00/08162, published February 17, 2000], which carries the 35S promoter, using an appropriate linker. The DNA containing the RTCS ORF is amplified through PCR by using specific primer sets and the cDNA as a template. The fragment is then digested with appropriate restriction enzymes of the vector between the 35S promoter and the transcription terminator. The appropriate orientation of the insert is confirmed by sequencing.

The construct with the ubiquitin promoter is made as follows: the transcription termination element is released from the clone by digestion with the appropriate restriction enzymes digestion. The fragment is ligated to BamHI and Notl restriction sites of SK-ubi (Bbsl), which carries the ubiquitin promoter (maize *Ubi-*1 promoter, Christensen and Quail (1996) Transgenic Res. 5: 213-218), using the an appropriate linker. The DNA containing the RTCS ORF is amplified through PCR by using a specific primer set and the cDNA as a template. The fragment is then digested with appropriate restriction enzymes and inserted between the ubiquitin promoter and the transcription terminator.

Plasmid pML103 has been deposited under the terms of the Budapest Treaty at ATCC (American Type Culture Collection, 10801 University Blvd., Manassas, VA 20110-2209), and bears accession number ATCC 97366. The DNA segment from pML103 contains a 1.05 kb Sall-Ncol promoter fragment of the maize 27 kD zein gene [Prat et al. (1987) Gene 52:51-49; Gallardo et al. (1988) PlantSci. 54:211-2811] and a 0.96 kb Smal-Sall fragment from the 3' end of the maize 10 kD zein gene in the vector pGem9Zf(+) (Promega). Vector and insert DNA can be ligated at 15°C overnight, essentially as described (Maniatis). The ligated DNA may then be used to transform *E. coli* XL1-Blue (Epicurian Coli XL-1 Blue™; Stratagene). Bacterial transformants can be screened by restriction enzyme digestion of plasmid DNA and limited nucleotide sequence analysis using the dideoxy chain termination method (Sequenase™ DNA Sequencing Kit; U.S. Biochemical). The resulting plasmid construct would comprise a recombinant DNA construct encoding, in the 5' to 3' direction, the maize 27 kD zein promoter, a cDNA fragment encoding the instant polypeptides, and the 10 kD zein 3'region.

The recombinant DNA construct described above can then be introduced into corn cells by the following procedure. Immature corn embryos can be dissected from developing caryopses derived from crosses of the inbred corn lines H99 and LH132. The embryos are isolated 10 to 11 days after pollination when they are 1.0 to 1.5 mm long. The embryos are then placed with the axis-side facing down and in contact with agarose-solidified N6 medium (Chu et al. (1975) Sci. Sin. Peking 18:659-668). The embryos are kept in the dark at 27°C. Friable embryogenic callus consisting of undifferentiated masses of cells with somatic proembryoids and embryoids borne on suspensor structures proliferates from the scutellum of these immature embryos. The embryogenic callus isolated from the primary explant can be cultured on N6 medium and sub-cultured on this medium.every 2 to 3 weeks.

The plasmid, p35S/Ac (obtained from Dr. Peter Eckes, Hoechst Ag, Frankfurt, Germany) may be used in transformation experiments in order to provide for a selectable marker. This plasmid contains the *Pat* gene (see European Patent Publication 0 242 236) which encodes phosphinothricin acetyl transferase (PAT). The enzyme PAT confers resistance to herbicidal glutamine synthetase inhibitors such as phosphinothricin. The *pat* gene in p35S/Ac is under the control of the 35S promoter from Cauliflower Mosaic Virus (Odell et al. (1985) Nature 313:810-812) and the 3' region of the nopaline synthase gene from the T-DNA of the Ti plasmid of *Agrobacterium tumefaciens.*

The particle bombardment method (Klein et al. (1987) Nature 327:70-73) may be used to transfer genes to the callus culture cells. According to this method, gold particles (1 µm in diameter) are coated with DNA using the following technique. Ten µg of plasmid DNAs are added to 50 µL of a suspension of gold particles (60 mg per mL). Calcium chloride (50 µL of a 2.5 M solution) and spermidine free base (20 µL of a 1.0 M solution) are added to the particles. The suspension is vortexed during the addition of these solutions. After 10 minutes, the tubes are briefly centrifuged (5 sec at 15,000 rpm) and the supernatant removed. The particles are resuspended in 200 µL of absolute ethanol, centrifuged again and the supernatant removed. The ethanol rinse is performed again and the particles resuspended in a final volume of 30 µL of ethanol. An aliquot (5 µL) of the DNA-coated gold particles can be placed in the center of a Kapton™ flying disc (Bio-Rad Labs). The particles are then accelerated into the corn tissue with a Biolistic™ PDS-1000/He (Bio-Rad Instruments, Hercules CA), using a helium pressure of 1000 psi, a gap distance of 0.5 cm and a flying distance of 1.0 cm.

For bombardment, the embryogenic tissue is placed on filter paper over agarose-solidified N6 medium. The tissue is arranged as a thin lawn and covered a circular area of about 5 cm in diameter. The petri dish containing the tissue can be placed in the chamber of the PDS-1000/He approximately 8 cm from the stopping screen. The air in the chamber is then evacuated to a vacuum of 28 inches of Hg. The macrocarrier is accelerated with a helium shock wave using a rupture membrane that bursts when the He pressure in the shock tube reaches 1000 psi.

Seven days after bombardment the tissue can be transferred to N6 medium that contains bialophos (5 mg per liter) and lacks casein or proline. The tissue continues to grow slowly on this medium. After an additional 2 weeks the tissue can be transferred to fresh N6 medium containing bialophos. After 6 weeks, areas of about 1 cm in diameter of actively growing callus can be identified on some of the plates containing the bialophos-supplemented medium. These calli may continue to grow when sub-cultured on the selective medium.

Plants can be regenerated from the transgenic callus by first transferring clusters of tissue to N6 medium supplemented with 0.2 mg per liter of 2,4-D. After two weeks the tissue can be transferred to regeneration medium (Fromm et al. (1990) Bio/Techno/ogy 8:833-839).

### EXAMPLE 12

### Expression of Recombinant DNA Constructs in Dicot Cells

The 35S promoter of CaMV can be used to over-express and co-suppress the genes homologous to *RTCS* in dicot cells. For *RTCS* overexpression, the vector KS50 can be used to fuse the *RTCS* ORF to the 35S promoter. The *RTCS* ORF is amplified by PCR using a specific primer set. The amplified DNA fragment is digested with the appropriate restriction enzyme and ligated at the appropriate site of KS50. The correct orientation of the insert is determined by sequencing. KS50 (7,453 bp) is a derivative of pKS18HH (U.S. Patent No. 5,846,784) which contains a T7 promoter/T7 terminator controlling the expression of a hygromycin phosphotransferase (HPT) gene, as well as a 35S promoter/NOS terminator controlling the expression of a second HPT gene. KS50 has an insert at the Sal I site consisting of a 35S promoter (960 bp)/NOS terminator (700 bp) cassette taken from pAW28, with a Notl cloning site between the promoter and terminator.

Soybean embryos may then be transformed with the expression vector comprising sequences encoding the instant polypeptides. To induce somatic embryos, cotyledons, 3-5 mm in length dissected from surface sterilized, immature seeds of the soybean cultivar A2872, can be cultured in the light or dark at 26°C on an appropriate agar medium for 6-10 weeks. Somatic embryos, which produce secondary embryos, are then excised and placed into a suitable liquid medium. After repeated selection for clusters of somatic embryos, which multiplied as early, globular staged embryos, the suspensions are maintained as described below.

Soybean embryogenic suspension cultures can be maintained in 35 mL liquid media on a rotary shaker, 150 rpm, at 26°C with florescent lights on a 16:8 hour day/night schedule. Cultures are subcultured every two weeks by inoculating approximately 35 mg of tissue into 35 mL of liquid medium.

Soybean embryogenic suspension cultures may then be transformed by the method of particle gun bombardment (Klein et al. (1987) Nature (London) 327:70-73, U.S. Patent No. 4,945,050). A DuPont BioliStic™ PDS1000/HE instrument (helium retrofit) can be used for these transformations.

A selectable marker gene which can be used to facilitate soybean transformation is a recombinant construct composed of the 35S promoter from Cauliflower Mosaic Virus (Odell et al. (1985) Nature 313:810-812), the hygromycin phosphotransferase gene from plasmid pJR225 (from *E. coli*; Gritz et al:(1983) Gene 25:179-188) and the 3' region of the nopaline synthase gene from the T-DNA of the Ti plasmid of *Agrobacterium tumefaciens.* The seed expression cassette comprising the phaseolin 5' region, the fragment encoding the instant polypeptides and the phaseolin 3' region can be isolated as a restriction fragment. This fragment can then be inserted into a unique restriction site of the vector carrying the marker gene.

To 50 µL of a 60 mg/mL 1 µm gold particle suspension is added (in order): 5 µL DNA (1 µg/µL), 20 µL spermidine (0.1 M), and 50 µL CaCl₂ (2.5 M). The particle preparation is then agitated for three minutes, spun in a microfuge for 10 seconds and the supernatant removed. The DNA-coated particles are then washed once in 400 µL 70% ethanol and resuspended in 40 µL of anhydrous ethanol. The DNA/particle suspension can be sonicated three times for one second each. Five µL of the DNA-coated gold particles are then loaded on each macro carrier disk.

Approximately 300-400 mg of a two-week-old suspension culture is placed in an empty 60x15 mm petri dish and the residual liquid removed from the tissue with a pipette. For each transformation experiment, approximately 5-10 plates of tissue are normally bombarded. Membrane rupture pressure is set at 1100 psi and the chamber is evacuated to a vacuum of 28 inches mercury. The tissue is placed approximately 3.5 inches away from the retaining screen and bombarded three times. Following bombardment, the tissue can be divided in half and placed back into liquid and cultured as described above.

Five to seven days post bombardment, the liquid media may be exchanged with fresh media, and eleven to twelve days post bombardment with fresh media containing 50 mg/mL hygromycin. This selective media can be refreshed weekly. Seven to eight weeks post bombardment, green, transformed tissue may be observed growing from untransformed, necrotic embryogenic clusters. Isolated green tissue is removed and inoculated into individual flasks to generate new, clonally propagated, transformed embryogenic suspension cultures. Each new line may be treated as an independent transformation event. These suspensions can then be subcultured and maintained as clusters of immature embryos or regenerated into whole plants by maturation and germination of individual somatic embryos.

### EXAMPLE 13

### Identification of protein sequences specific to RTCS and RTCS homologs

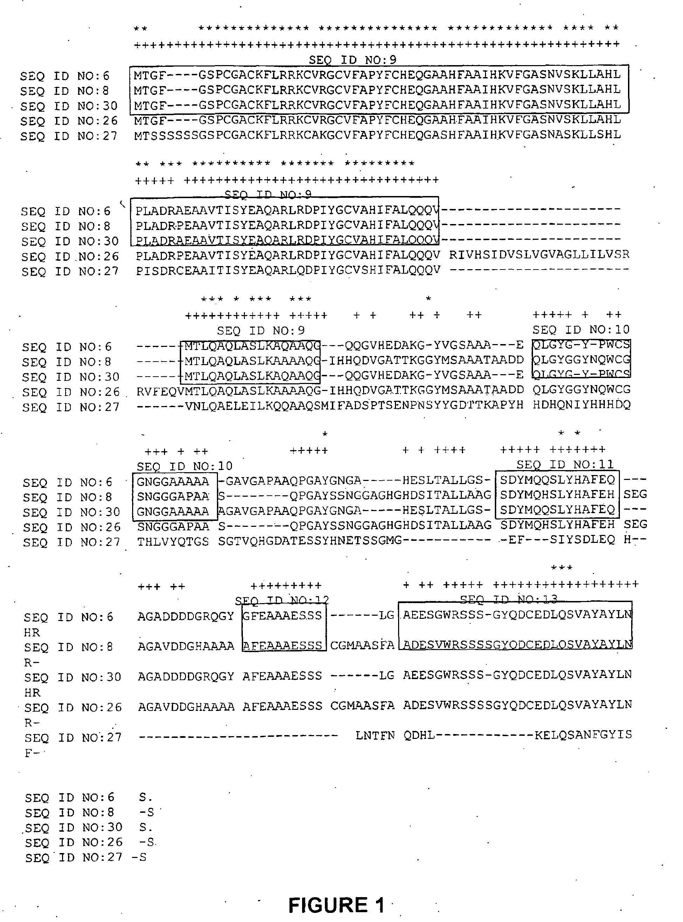

LOB domain proteins comprise a family of genes involved in lateral organ boundary formation and all share a conserved amino acid sequence stretch of about 100 residues in the N-terminal regions of the proteins belonging to this family (Shuai et al. (2002) Plant Physiology : 129: 747-761). Figure 1 shows an alignment of the maize RTCS from inbred Mo17 (SEQ ID NO:6), a deduced RTCS homolog from rice (SEQ ID NO:8), maize RTCS from inbred B73 (SEQ ID NO:30), rice gi 27261472 (SEQ ID NO:26) and *Arabidopsis* gi: 22331847 (SEQ ID NO:27). The boxed residues are conserved motifs unique to RTCS proteins.

## Claims

1. An isolated polynucleotide comprising:
(a) a nucleotide sequence encoding a polypeptide required for proper root formation, wherein the absence of or altered levels of the polypeptide in a plant results in the elimination of or altered levels of formation of one or more of the nodal roots, wherein the polypeptide has an amino acid sequence of at least 70% sequence identity, based on the Clustal V method of alignment, when compared to one of SEQ ID NO:6, 8, 30, or 38; or
(b) a complement of the nucleotide sequence, wherein the complement and the nucleotide sequence consist of the same number of nucleotides and are 100% complementary.

2. The polynucleotide of Claim 1, wherein the amino acid sequence of the polypeptide has at least 75% sequence identity, based on the Clustal V method of alignment, when compared to one of SEQ ID NO:6, 8,30, or 38.

3. The polynucleotide of Claim 1, wherein the amino acid sequence of the polypeptide has at least 80% sequence identity, based on the Clustal V method of alignment, when compared to one of SEQ ID NO:6, 8 ,30, or 38.

4. The polynucleotide of Claim 1, wherein the amino acid sequence of the polypeptide has at least 85% sequence identity, based on the Clustal V method of alignment, when compared to one of SEQ ID NO:6, 8 ,30, or 38.

5. The polynucleotide of Claim 1, wherein the amino acid sequence of the polypeptide has at least 90% sequence identity, based on the Clustal V method of alignment, when compared to one of SEQ ID NO:6, 8 ,30, or 38.

6. The polynucleotide of Claim 1, wherein the amino acid sequence of the polypeptide has at least 95% sequence identity, based on the Clustal V method of alignment, when compared to one of SEQ ID NO:6, 8 ,30, or 38.

7. The polynucleotide of Claim 1, wherein the amino acid sequence of the polypeptide has at least 99% sequence identity, based on the Clustal V method of alignment, when compared to SEQ ID NO: 6, 8 ,30, or 38.

8. The polynucleotide of Claim 1, wherein the amino acid sequence of the polypeptide comprises one of SEQ ID NO:6, 8 ,30, or 38.

9. The polynucleotide of Claim 1 wherein the nucleotide sequence comprises one of SEQ ID NO:5, 7,29 or 37.

10. The isolated polynucleotide of Claim 1, wherein the nucleotide sequence comprises a sequence which encodes at least two motifs selected from group consisting of SEQ ID NOs:9, 10, 11, 12 and 13, wherein said motif is a substantially conserved subsequence.

11. A functionally equivalent subfragment of the isolated polynucleotide of Claim 1, wherein said subfragment is useful in antisense inhibition or co-suppression of expression of a nucleic acid sequence encoding the polypeptide of Claim 1.

12. An isolated nucleic acid fragment comprising a promoter consisting essentially of SEQ ID NO:2, 3 or 4, or a substantially similar and functionally equivalent subfragment of said promoter.

13. A recombinant DNA construct comprising the isolated polynucleotides of Claim 1 or a functionally equivalent subfragment thereof, operably linked to at least one regulatory sequence.

14. The recombinant DNA construct of Claim 13, wherein said at least one regulatory sequence comprises the promoter of Claim 12.

15. A plant comprising in its genome the recombinant DNA construct of Claim 13.

16. A seed obtained from the plant of Claim 15, wherein the seed comprises in its genome the recombinant DNA construct of Claim 13.

17. The plant of Claim 15, wherein said plant is selected from the group consisting of rice, corn, sorghum, millet, rye, soybean, canola, wheat, barley, oat, beans, and nuts.

18. Transformed plant tissue or plant cell comprising the recombinant DNA construct of Claim 13.

19. A method of altering root structure during plant development, comprising:
(a) transforming a plant with the recombinant DNA construct of Claim 13;
(b) growing the transformed plant under conditions suitable for the expression of the recombinant DNA construct; and
(c) selecting those transformed plants having altered root structure.

20. A method to isolate nucleic acid fragments encoding polypeptides associated with altering root structure during plant development, comprising:
(a) comparing SEQ ID NOs:6,8 30, or 38 with other polypeptide sequences associated with altering root structure during plant development;
(b) identifying the conserved sequences(s) of 4 or more amino acids obtained in step (a);
(c) making region-specific nucleotide probe(s) or oligomer(s) based on the conserved sequences identified in step (b); and
(d) using the nucleotide probe(s) or oligomer(s) of step (c) to isolate sequences associated with altering root structure during plant development by sequence dependent protocols.

## Patentansprüche

1. Isoliertes Polynukleotid, umfassend:
(a) eine Nukleotidsequenz, kodierend für ein Polypeptid, das für eine angemessene Wurzelbildung erforderlich ist, worin die Abwesenheit der oder veränderter Spiegel des Polypeptids in einer Pflanze zur Eliminierung der oder veränderter Spiegel zur Bildung von einer oder mehr der Knotenwurzeln führt, worin das Polypeptid, bezogen auf das Clustal V-Alignmentverfahren, eine Aminosäuresequenz von mindestens 70 %iger Sequenzidentität aufweist, wenn sie mit einer von SEQ ID NO: 6, 8, 30 oder 38 verglichen wird; oder
(b) ein Komplement der Nukleotidsequenz, worin das Komplement und die Nukleotidsequenz aus der gleichen Nukleotidzahl bestehen und 100 % komplementär sind.

2. Polynukleotid nach Anspruch 1, worin die Aminosäuresequenz des Polypeptids, bezogen auf das Clustal V-Alignmentverfahren, eine mindestens 75 %ige Sequenzidentität aufweist, wenn sie mit einer von SEQ ID NO: 6, 8, 30 oder 38 verglichen wird.

3. Polynukleotid nach Anspruch 1, worin die Aminosäuresequenz des Polypeptids, bezogen auf das Clustal V-Alignmentverfahren, eine mindestens 80 %ige Sequenzidentität aufweist, wenn sie mit einer von SEQ ID NO: 6, 8, 30 oder 38 verglichen wird.

4. Polynukleotid nach Anspruch 1, worin die Aminosäuresequenz des Polypeptids, bezogen auf das Clustal V-Alignmentverfahren, eine mindestens 85 %ige Sequenzidentität aufweist, wenn sie mit einer von SEQ ID NO: 6, 8, 30 oder 38 verglichen wird.

5. Polynukleotid nach Anspruch 1, worin die Aminosäuresequenz des Polypeptids, bezogen auf das Clustal V-Alignmentverfahren, eine mindestens 90 %ige Sequenzidentität aufweist, wenn sie mit einer von SEQ ID NO: 6, 8, 30 oder 38 verglichen wird.

6. Polynukleotid nach Anspruch 1, worin die Aminosäuresequenz des Polypeptids, bezogen auf das Clustal V-Alignmentverfahren, eine mindestens 95 %ige Sequenzidentität aufweist, wenn sie mit einer von SEQ ID NO: 6, 8, 30 oder 38 verglichen wird.

7. Polynukleotid nach Anspruch 1, worin die Aminosäuresequenz des Polypeptids, bezogen auf das Clustal V-Alignmentverfahren, eine mindestens 99 %ige Sequenzidentität aufweist, wenn sie mit einer von SEQ ID NO: 6, 8, 30 oder 38 verglichen wird.

8. Polynukleotid nach Anspruch 1, worin die Aminosäuresequenz des Polypeptids eine von SEQ ID NO: 6, 8, 30 oder 38 umfasst.

9. Polynukleotid nach Anspruch 1, worin die Nukleotidsequenz eine von SEQ ID NO: 5, 7, 29 oder 37 umfasst.

10. Isoliertes Polynukleotid nach Anspruch 1, worin die Nukleotidsequenz eine Sequenz umfasst, die für mindestens zwei Motive kodiert, die aus der Gruppe ausgewählt sind, bestehend aus SEQ ID NO: 9, 10, 11, 12 und 13, worin das Motiv eine im Wesentlichen konservierte Subsequenz darstellt.

11. Funktionell äquivalentes Subfragment des isolierten Polynukleotids nach Anspruch 1, worin das Subfragment bei der Antisense-Inhibition oder Cosuppression der Expression einer für das Polypeptid nach Anspruch 1 kodierenden Nukleinsäuresequenz nützlich ist.

12. Isoliertes Nukleinsäurefragment, das einen Promotor umfasst, bestehend im Wesentlichen aus SEQ ID NO: 2, 3 oder 4, oder einem im Wesentlichen ähnlichen und funktionell äquivalenten Subfragment des Promotors.

13. Rekombinantes DNA-Konstrukt, umfassend das isolierte Polynukleotid nach Anspruch 1 oder ein funktionell äquivalentes Subfragment davon, das operativ mit mindestens einer Regulatorsequenz verknüpft ist.

14. Rekombinantes DNA-Konstrukt nach Anspruch 13, worin die mindestens eine Regulatorsequenz den Promotor nach Anspruch 12 umfasst.

15. Pflanze, umfassend in ihrem Genom das rekombinante DNA-Konstrukt nach Anspruch 13.

16. Samen, erhalten aus der Pflanze nach Anspruch 15, worin der Samen in seinem Genom das rekombinante DNA-Konstrukt nach Anspruch 13 umfasst.

17. Pflanze nach Anspruch 15, worin die Pflanze aus der Gruppe ausgewählt ist, bestehend aus Reis, Mais, Sorghum, Hirse, Roggen, Sojabohne, Raps, Weizen, Gerste, Hafer, Bohnen und Nüssen.

18. Transformierte(s) Pflanzengewebe oder Pflanzenzelle, umfassend das rekombinante DNA-Konstrukt nach Anspruch 13.

19. Verfahren zum Verändern der Wurzelstruktur während der Entwicklung der Pflanze, umfassend:
(a) Transformieren einer Pflanze mit dem rekombinanten DNA-Konstrukt nach Anspruch 13;
(b) Züchten der transformierten Pflanze unter Bedingungen, die für die Expression des rekombinanten DNA-Konstrukts geeignet sind; und
(c) Auswählen der transformierten Pflanzen, die eine veränderte Wurzelstruktur aufweisen.

20. Verfahren zum Isolieren von Nukleinsäurefragmenten, die für Polypeptide kodieren, die mit dem Verändern der Wurzelstruktur während der Entwicklung der Pflanze assoziiert sind, umfassend:
(a) Vergleichen von SEQ ID NO: 6, 8, 30 oder 38 mit anderen Polypeptidsequenzen, die mit dem Verändern der Wurzelstruktur während der Entwicklung der Pflanze assoziiert sind;
(b) Identifizieren der konservierten Sequenz(en) von 4 oder mehr Aminosäuren, die in Schritt (a) erhalten wurden;
(c) Herstellen einer/von regionsspezifischen Nukleotidsonde(n) oder einem/von regionsspezifischen Oligomer(en) bezogen auf die in Schritt (b) identifizierten konservierten Sequenzen; und
(d) Verwenden der Nukleotidsonde(n) oder des Oligomers/der Oligomere von Schritt (c) zum Isolieren von Sequenzen, die mit dem Verändern der Wurzelstruktur während der Entwicklung der Pflanze assoziiert sind, mittels sequenzabhängiger Protokolle.

## Revendications

1. Polynucléotide isolé comprenant:
(a) une séquence de nucléotides codant un polypeptide nécessaire à la formation correcte des racines, où l'absence de/ou des niveaux modifiés du polypeptide dans une plante résulte en l'élimination de/ou des niveaux modifiés de formation d'une ou plusieurs racines nodales, où le polypeptide a une séquence d'acides aminés d'au moins 70% d'identité de séquence, sur la base de la méthode d'alignement Clustal V, lorsqu'elle est comparée à l'une des SEQ ID NO: 6, 8, 30 ou 38; ou
(b) un complément de la séquence de nucléotides, où le complément et la séquence de nucléotides consistent en le même nombre de nucléotides et sont complémentaires à 100%.

2. Polynucléotide selon la revendication 1, dans lequel la séquence d'acides aminés du polypeptide a au moins 75% d'identité de séquence, sur la base de la méthode d'alignement Clustal V, lorsqu'elle est comparée à l'une des SEQ ID NO: 6, 8, 30 ou 38.

3. Polynucléotide selon la revendication 1, dans lequel la séquence d'acides aminés du polypeptide a au moins 80% d'identité de séquence, sur la base de la méthode d'alignement Clustal V, lorsqu'elle est comparée à l'une des SEQ ID NO: 6, 8, 30 ou 38.

4. Polynucléotide selon la revendication 1, dans lequel la séquence d'acides aminés du polypeptide a au moins 85% d'identité de séquence, sur la base de la méthode d'alignement Clustal V, lorsqu'elle est comparée à l'une des SEQ ID NO: 6, 8, 30 ou 38.

5. Polynucléotide selon la revendication 1, dans lequel la séquence d'acides aminés du polypeptide a au moins 90% d'identité de séquence, sur la base de la méthode d'alignement Clustal V, lorsqu'elle est comparée à l'une des SEQ ID NO: 6, 8, 30 ou 38.

6. Polynucléotide selon la revendication 1, dans lequel la séquence d'acides aminés du polypeptide a au moins 95% d'identité de séquence, sur la base de la méthode d'alignement Clustal V, lorsqu'elle est comparée à l'une des SEQ ID NO: 6, 8, 30 ou 38.

7. Polynucléotide selon la revendication 1, dans lequel la séquence d'acides aminés du polypeptide a au moins 99% d'identité de séquence, sur la base de la méthode d'alignement Clustal V, lorsqu'elle est comparée à l'une des SEQ ID NO: 6, 8, 30 ou 38.

8. Polynucléotide selon la revendication 1, dans lequel la séquence d'acides aminés du polypeptide comprend l'une des SEQ ID NO: 6, 8, 30 ou 38.

9. Polynucléotide selon la revendication 1, dans lequel la séquence de nucléotides comprend l'une des SEQ ID NO: 5, 7, 29 ou 37.

10. Polynucléotide isolé selon la revendication 1, dans lequel la séquence de nucléotides comprend une séquence qui code au moins deux motifs sélectionnés parmi le groupe consistant en la SEQ ID NO: 9, 10, 11, 12 et 13, où ledit motif est une sous-séquence sensiblement conservée.

11. Sous-fragment fonctionnellement équivalent du polynucléotide isolé selon la revendication 1, où ledit sous-fragment est utile dans inhibition anti-sens ou la co-suppression de l'expression d'une séquence d'acide nucléique codant le polypeptide selon la revendication 1.

12. Fragment d'acide nucléique isolé comprenant un promoteur consistant essentiellement en la SEQ ID NO: 2, 3 ou 4 ou un sous-fragment sensiblement semblable et fonctionnellement équivalent dudit promoteur.

13. Produit de synthèse d'ADN recombinant comprenant Polynucléotide isolé selon la revendication 1 ou un sous-fragment fonctionnellement équivalent du même, lié de manière opérationnelle à au moins une séquence régulatrice.

14. Produit de synthèse d'ADN recombinant selon la revendication 13, dans lequel ladite au moins une séquence régulatrice comprend le promoteur selon la revendication 12.

15. Plante comprenant le produit de synthèse d'ADN recombinant selon la revendication 13 dans son génome.

16. Graine obtenue de la plante selon la revendication 15, où la graine comprend le produit de synthèse d'ADN recombinant selon la revendication 13 dans son génome.

17. Plante selon la revendication 15, où ladite plante est sélectionnée parmi le groupe consistant en riz, maïs, sorgho, millet, seigle, soja, colza, blé, orge, avoine, haricots et noix.

18. Tissu végétal ou cellule végétale transformé(e) comprenant le produit de synthèse d'ADN recombinant selon la revendication 13.

19. Méthode de modification de la structure des racines au cours du développement d'une plante, comprenant les étapes consistant à :
(a) transformer une plante avec le produit de synthèse d'ADN recombinant selon la revendication 13;
(b) faire pousser la plante transformée dans des conditions qui conviennent à l'expression du produit de synthèse d'ADN recombinant; et
(c) sélectionner ces plantes transformées possédant une structure de racines modifiée.

20. Méthode d'isolement de fragments d'acide nucléique codant des polypeptides associés à la modification de la structure des racines pendant le développement d'une plante, comprenant les étapes consistant à :
(a) comparer les SEQ ID NO: 6, 8, 30 ou 38 à d'autres séquences de polypeptides associés à la modification de la structure des racines pendant le développement d'une plante;
(b) identifier la/les séquence(s) conservée(s) de 4 acides aminés ou plus obtenus à l'étape (a);
(c) préparer une/des sonde(s) nucléotidique(s) ou oligomère(s) région-spécifique(s) sur la base des séquences conservées identifiées à l'étape (b); et
(d) utiliser la/les sonde(s) nucléotidique(s) ou oligomère(s) de l'étape (c) pour isoler des séquences associées à la modification de la structure des racines pendant le développement d'une plante, avec des protocoles dépendants des séquences.
